(19) Europäisches Patentamt
European Patent Office
Office européen des brevets

(11) **EP 4 721 566 A1**

(12) **EUROPEAN PATENT APPLICATION**

(43) Date of publication:
08.04.2026 Bulletin 2026/15

(21) Application number: 24383080.9

(22) Date of filing: 07.10.2024

(51) International Patent Classification (IPC):
*A01N 63/22* (2020.01)    *A01P 21/00* (2006.01)
*C12N 1/20* (2026.01)

(52) Cooperative Patent Classification (CPC):
(C-Sets available)
**A01N 63/22; A01P 21/00; C12N 1/20; C12N 1/205;**
C12R 2001/07                                    (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Kimitec Biogroup S.L**
**04738 Vícar Almeria (ES)**

(72) Inventors:
• **Torres Cortés, Gloria**
  **04378 Vicar, Almeria (ES)**

• **Velasco, Francisco**
  **04378 Vicar, Almeria (ES)**
• **Macias Zabala, Clara**
  **04378 Vicar, Almeria (ES)**
• **Guzmán García, Sofia**
  **04378 Vicar, Almeria (ES)**
• **Ramírez Santos, Adrián**
  **04378 Vicar, Almeria (ES)**

Remarks:
The complete document including Reference
Table(s) and the Sequence Listing(s) can be
downloaded from the EPO website

(54) **MICROBIAL COMPOSITION BASED ON BACILLUS INFANTIS STRAIN FOR AGRICULTURAL USE**

(57)    Disclosed herein is a strain of *Bacillus infantis* deposited under number CECT 30759. Also, a microbial composition and process for the preparation thereof. Particularly, a microbial composition based on *Bacillus infantis* strain used as plant growth promotion bacteria (PGPB), more specifically as a biological fixing nitrogen. In addition, the present invention discloses a method for reducing the total amount of nitrogen fertilizer required by crop.

Figure 8

(52) Cooperative Patent Classification (CPC): (Cont.)

C-Sets
**A01N 63/22, A01N 63/20**

(52) Cooperative Patent Classification (CPC): (Cont.)

C-Sets
**A01N 63/22, A01N 63/20**

**Description**

**Reference to a sequence listing**

This application contains a Sequence Listing in computer readable form. The contents of the electronic sequence listing in computer readable form (Sequence_listing.xml; 15,694 bytes; created September 30, 2024) is hereby incorporated by reference.

**Technical Field**

The present invention is related to microbial composition and a method for agricultural process and use. Particularly, compositions based on *Bacillus infantis* strain used as plant growth promotion bacteria (PGPB), more specifically as a biological fixing nitrogen.

**Background Art**

World food demand continues to increase along with global population. According to the European Commission, the population in the world was growing at a 2 % annual rate from the sixties to the eighties. Since then, world population increased by 2.5 billion people to reach 7.7 billion worldwide in 2019. Population growth is and will remain a driver for food demand in the future, even though rates closer to 1 %. Still, it is not the sole driver, as per capita consumption has also been increasing at a rapid pace.

As a consequence, there is a need to increase the numbers of land available for agriculture that can face the challenge of change climate without decreasing of the rate of production. In order to achieve this goal shall be developed inputs and techniques that can increase food production. Also, these innovations must be environmental-friendly decreasing the use of potentially harmful pesticides.

The need of nitrogen for the development of plants is essential for increasing the level of production in crops. Nitrogen is one of the macronutrients which is required in large amount for plant metabolism and growth act as a primary nutrient for plants. It is absorbed in Ammonium ($NH_4+$), Nitrate ($NO_3-$) ions forms. Nitrogen is a major component of chlorophyll, the compound by which plants use sunlight energy for photosynthesis to produce sugars from water and carbon dioxide. Nitrogen produces early growth and also results in delay in maturity in plants. Nitrogen is the element which is not directly available to plants from atmosphere and earth's crust.

For all the above-mentioned reasons, application of nitrogen fertilizer has become an essential practice in modern agriculture as it is vital to maintain competitive crop productivity. However, excessive use of nitrate fertilizers can lead to health and environmental issues (Zayed 2023). Also, the nitrogen fertilizers comprise as main components: Urea (EP0376853, EP4247775, and EP1379558) being known the adverse effect of urea fertilizer on seed germination and seedling growth in soil (Krogmeier 1989); ammonium chloride (CN101565125, EP4330263) that aggravates salt stress in plants (Liu XX 2020); or sulfuric acid (EP1114009) that is very corrosive and would badly burn any plants, birds or land animals exposed to it. (Lowell 1978). Therefore, alternative strategies, such as establishing nitrogen fixation in plants through diazotrophic microbiota, have been explored to reduce reliance on synthetic fertilizers (Solanki 2020).

The symbiosis of microbial consortia-plant involves for use in agricultural or biodegradation applications is known. Microorganisms play an important role in the nitrogen fixation that convert atmospheric nitrogen ($N_2$) which is inert form into usable ammonia ($NH_3$). In the state of art can be found biological nitrogen fixation compositions that can become toxic for containing metal ions (US 9,580,363). For this reason, it is necessary to develop new composition used to fix nitrogen that can be environmental ecofriendly and effective.

*Bacillus sp.* has been used in agriculture for controlling a plurality of plant diseases (CN106085898), nematicide (Tran 2018), for improving available nutrients in rhizosphere soil of crops, (CN107164279), for recovering contaminated soil (KR10-2022-0162897), (Desai 2008), siderophore-producing bacteria (Tang 2021). Also, biodegradation of different compounds such as azo dye (Ankita 2012), polyhydroxyalkanoates (Volova 2011) and crude oil, (Rodrigo 2017). Other applications described for recycling on-farm wastes and food production (Girish 2018), for producing compounds with algicidal activity (Sun 2012), cosmetics (Dahal 2019) and carotenoids (Khaneja 2009). However, *Bacillus subtilis* is not described in the state of art having capability of nitrogen fixation.

In microbiology, the phyllosphere is the total above-ground surface of a plant when viewed as a habitat for microorganisms. The phyllosphere can be further subdivided into the caulosphere (stems), phylloplane (leaves), anthosphere (flowers), and carposphere (fruits). The below-ground microbial habitats (i.e. the thin-volume of soil surrounding root or subterranean stem surfaces) are referred to as the rhizosphere. Leaf surfaces (phyllospheres) have been shown to provide appropriate conditions for colonization by microorganisms including diazotrophic bacteria that are able to fix atmospheric nitrogen (Fürnkranz 2008) improving the nitrogen fixation compared with the associated bacteria to the roots.

## Summary of invention

The present invention resolves the following problems: lack of the mineral nutrition in plants related to biological fixing nitrogen, as result an increasing the production in wheat, corn or lettuce crops; also increasing the availability of phytohormones in plants, auxins and cytokinins, acting as a biostimulant providing a direct effect on the growth of plant and production of crops.

The present invention is a strain of Bacillus *infantis* deposited with Spanish type culture collection (CECT) under Budapest treaty on November 30th, 2022, and assigned deposit number CECT 30759 characterized by promoting the growth in plant and more surprisingly to have capability of fixing nitrogen.

In some embodiment, the strain according to the present invention their nucleotides sequences share at least 97% identity to SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

Other aspect of the present invention describes a microbial composition for agricultural use. In some embodiments, a microbial composition of the present invention includes the strain *Bacillus infantis* CECT 30759; at least one microorganism used in agriculture; and one or more compounds used in formulation of agricultural products.

In the present invention, the microorganism used in agriculture is referred to bioinoculants comprised of living or dormant microbes that are able to promote growth and development in plant and have great potential not only for enhancing plant yield but also for remediation of degraded soils. Bioinoculants are cost-effective and environmental-friendly approaches in agriculture.

Microorganisms used in agriculture such as referred in the present invention are bacteria, fungi, yeast, and algae selected from but not limited to the group consisting of Table 1 shown below.

### TABLE 1: Microorganisms used in agriculture

| Microorganism | Plant | Applications |
|---|---|---|
| *Acetobacter sp* | *Saccharum officinarum* | Biofertilization for sugarcane. |
| *Acidisoma sp.* | *Quercus sp.* | Hydrolyzing Cellulose and Producing Poly-3-hydroxybutyrate |
| *Acinetobacter sp.* | *Zea mays* | Increasing tolerance to Cu; enhanced chlorophyll content increasing Cu concentration in tissues; indole-3-acetic acid. Synthesis; production of siderophores solubilization of Cu and P. |
| | *Saccharum sp.* | Under salt stress enhanced number of leaves, fresh, dry weight, and germination ratio; $N_2$ assimilation. |
| *Agrobacterium sp.* | *Phaseolus vulgaris* | Increasing nodule formation; higher plant biomass; Enhancing content of P, polyphenols, and flavonoids in grains, changes in the structure of the microbial community. |
| *Alternaria sp.* | *Capsicum annum* | Enhancing number of leaves, flowers, dry, and fresh weight. |
| *Anabaena sp.* | *Oryza sativa* | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| | *Lycopersicon esculentum* | Protection against *Fusarium* wilt (F. *oxysporum)* with significant enhancement of growth, yield, and fruit quality. Increasing N, P, and Zn concentration. Increasing activity of defense enzymes (phenylalanine ammonia-lyase, polyphenol oxidase), increased activity of chitosanase, and β-1,3-glucanase. |
| | *Triticum aestivum* | Enhancing biomass. Nitrogen-fixing potential. Higher endoglucanase activity. |
| | *Zea mays, Triticum aestivum* | Enhancing biomass, uptake of Fe and P, and higher chlorophyll content under iron-stress and siderophores production. |
| *Arthrobacter sp.* | *Lycopersicon esculentum* | Enhancing seed germination ratio, seedling length, and dry and fresh weight under salt stress. |

(continued)

| Microorganism | Plant | Applications |
|---|---|---|
| *Aspergillus sp.* | *Phasoleus vulgaris* | Increasing biomass. Production of IAA, amino cyclopropane carboxylate deaminase, siderophores, protease, amylase, pectinase, xylanase, and P solubilization. |
| | *Glycine max* | Salt stress alleviation with enhanced biomass, leaf area, chlorophyll content, and photosynthetic rate. Increasing isoflavones, proline, SA, and JA content and lower abscisic acid content and gibberellins production. |
| *Aulosira sp.* | *Oryza sativa* | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| *Azospirillum sp* | *Cicer arietinum* | Increasing resistance to *Ascochyta rabiei* via induction of plant defense-related genes. |
| | *Triticum aestivum* | Improving germination, plant growth, higher chlorophyll content, and improving membrane stability under salt stress. Increasing production of superoxide dismutase and osmolytes |
| *Azotobacter sp.* | *Zea mays* | Increasing shoot dry weight, chlorophyll content, and N, P, Fe concentration under drought stress and production of siderophore. |
| | *Gossypium hirsutum* | Increasing rate of seedling emergence, biomass, and N uptake and IAA synthesis. |
| *Bacillus sp* | *Lycopersicon esculentum* | Enhancing seed germination ratio, seedling length, and dry and fresh weight under salt stress. |
| | *Zea mays* | Increasing tolerance to salt stress, enhanced content of chlorophyll, soluble sugars, and glutathione, higher peroxidase/catalase activity. |
| | *Gossypium hirsutum* | Increasing rate of seedling emergence, biomass, and N uptake and IAA synthesis. |
| *Bradyrhizobium sp* | *Oryza sativa* | Increasing yield and uptake of N, P, K, and Fe. improved seed vigor, dry biomass, and leaf area with faster seedling emergence. IAA synthesis. |
| *Brevibacillus sp* | *Gossypium hirsutum* | Plant growth-promoting rhizobacteria, biocontrol agent of plant disease combat, and soil bioremediation to remove heavy metals. |
| *Burkholderia sp.* | *Triticum aestivum* | Improving water content and CO2 assimilation rate, water use efficiency, chlorophyll content, and higher yield under drought stress. Improving ionic balance, antioxidant levels, higher N, P, K, and protein content in grain |
| | *Lycopersicum esculentum* | Increasing yield and N-fixation and P solubilization |
| *Calothrix sp.* | *Oryza sativa* | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| | *Triticum aestivum* | Enhancing biomass. Nitrogen-fixing potential. Higher endoglucanase activity |
| *Candida sp* | *Oryza sativa* | Production plant growth regulators and mobilizes insoluble phosphate. |

(continued)

| Microorganism | Plant | Applications |
|---|---|---|
| Chlorella sp. | Cucumis sativus | Protection against anthracnose (Colletotrichum orbiculare) via the induction of SAR. |
| | Zea mays | Enhancing chlorophyll, P, and K content. Improving biomass. |
| | Telfairia occidentalis | Enhancing germination ratio. Higher number of leaves and yield. Improving chlorophyll, carbohydrates, proteins, and lipid content. |
| | Lycopersicon esculentum | Enhancing biomass and root length. |
| Clitocybe sp. | Brassica napus | Enhancing root and shoot growth, number of leaves, and biomass. Production of IAA. |
| Clostridium sp. | Pisum sativum L | Fungicidal Activity. Enhancing biomass and root length |
| Collybia sp. | Brassica napus | Enhancing root and shoot growth, number of leaves, and biomass. Production of IAA. |
| Cyathus sp | Brassica napus | Enhancing root and shoot growth, number of leaves, and biomass. Production of IAA. |
| Ensifer sp. | Vitis vinifera | Enhancing plant height and dry weight. higher VOCs content in roots. |
| Enterobacter sp. | Spinacia oleracea | Protection against Fusarium wilt (Fusarium oxysporum) |
| Frankia sp. | Casuarina glauca, Casuarina equisetifolia | Salt stress alleviation, higher dry biomass, chlorophyll, and proline content |
| Funneliformis sp. | Vitis vinifera | Enhancing plant height and dry weight. higher VOCs content in roots. |
| Fusarium sp. | Cucumis sativus | Protection against anthracnose (Colletotrichum orbiculare) and damping off (Rhizoctonia solani). Enhancing shoot dry weigh |
| | Glycine max | Salt stress alleviation with increased shoot length, protein content, carotenoid, SA, and enhanced SOD activity. Decreasing ABA level and lipid peroxidation level. |
| | Allium cepa | Protection against white rot disease (Sclerotium cepivorum) with enhanced plant height, dry weight, and bulb perimeter. Enhancing levels of peroxidase and polyphenol oxidase. Upregulation of plant defense genes (PR1, PR2). |
| Glomus sp. | Cucumis sativus | Protection against anthracnose (Colletotrichum orbiculare) and damping off (Rhizoctonia solani). Enhancing shoot dry weigh |
| | Olea europaea | Enhancing yield, dry weight, height, stem diameter, and root length. |
| Hapalosiphon sp. | Oryza sativa | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| Hebeloma sp. | Brassica napus | Enhancing root and shoot growth, number of leaves, and biomass. Production of IAA. |
| Humicola sp. | Glycine max | Salt stress alleviation with increased shoot length, protein content, carotenoid, SA, and enhanced SOD activity. Decreasing ABA level and lipid peroxidation level |
| Laccaria sp. | Brassica napus | Enhancing root and shoot growth, number of leaves, and biomass. Production of IAA. |

(continued)

| Microorganism | Plant | Applications |
|---|---|---|
| *Lactobacillus sp* | *Pyrus sp, Vitis vinifera, Prunus sp.* | Plant growth, biocontrol and bioremediation. |
| *Lecanicillium sp.* | *Elettaria cardamomum* | Enhancing shoot and root length, biomass, and number of leaves. Higher chlorophyll content. Production of IAA, ammonia, siderophores, and cell-wall degrading enzymes. P and Zn solubilization. |
| *Metarhizium sp.* | *Zea mays, Phaseolus vulgaris, Glycine max* | Enhancing plant height and biomass and N content in roots (*Z. mays*) and P in shoots (*P. vulgaris*). P solubilization and IAA production. |
| *Methylobacterium sp.* | *Arabidopsis thaliana, Solanum tuberosum* | Alleviation of salt stress of *A. thaliana,* with higher lateral roots density, number of leaves, and larger rosette diameter Reducing necrotic lesions and chlorosis in *S.tuberosum* infected with *P.infestans.* Production of IAA, P solubilization, biocontrol activity against *Phytophtora infestans, Botrytis cinerea,* and *Fusarium gramiearum.* |
| *Microcystis sp.* | *Oryza sativa* | Heavy metal (Cd) stress alleviation with decreased Cd accumulation, increasing translocation of Cd from root to shoot, and enhancing dry weight. |
| *Mortierella sp.* | *Triticum aestivum* | Enhancing fresh weight. Production of IAA, GA, and ACC deaminase |
| *Mucor sp.* | *Arabidopsis arenosa* | Heavy metal (Zn, Cd, Fe, Pb) stress alleviation with enhanced biomass, root hair growth, improved water, and P content. Upregulation of genes involved in nutrient acquisition *(HRS1, SPX1, MGD2),* and metal homeostasis *(MTPA2, ZIP7, IREG2, IRT2.* |
| | *Triticum aestivum, Oryza sativa* | Enhancing biomass and shoot/root length. Production of IAA and zeatin. |
| *Nostoc sp.* | *Zea mays* | Enhancing dry mass. Higher N content. Production of exopolysaccharide. |
| *Oscillatoria sp.* | *Oryza sativa* | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| *Paenibacillus sp.* | *Triticum, Cucumis sativus and Solanum lycopersicum,* | Plant growth-promoting rhizobacteria (PGPR), which competitively colonize plant roots and can simultaneously act as biofertilizers and as antagonists (biopesticides) of recognized root pathogens, such as bacteria, fungi, and nematodes. |
| *Pseudomonas sp.* | *Zea mays* | Increasing tolerance to Cu; enhanced chlorophyll content increasing Cu concentration in tissues; IAA Synthesis; production of siderophores solubilization of Cu and P. |
| | *Lycopersicum esculentum* | Increasing plant height, stem diameter, radical volume, dry biomass, and fruit yield. Production of IAA. |
| | *Solanum melongena* | Salt stress ameliorating effect, with increased dry weight, and seed germination. Higher superoxide dismutase activity in leaves. |
| | *Brassica napus* | Enhancing growth under salt stress, with a decrease in non-enzymatic antioxidants accumulation. Improving seed germination ratio, number of leaves, chlorophyll content, and dry weigh. |
| | *Triticum aestivum* | Salt stress alleviation with enhanced plant height, root length, and yield, and higher chlorophyll content. ACC deaminase production. |

(continued)

| Microorganism | Plant | Applications |
|---|---|---|
| Penicillium sp. | Pisum sativum | Increasing root dry weight, length, and P content in the shoot. |
| | Medicago lupulina, Lens culinaris, Triticum aestivum | In the plant is enhanced shoot growth and dry weight, and increased P uptake. |
| | Allium cepa | Protection against white rot disease (Sclerotium cepivorum) with enhanced plant height, dry weight, and bulb perimeter. Enhancing levels of peroxidase and polyphenol oxidase. Upregulation of plant defense genes (PR1, PR2). |
| Phoma sp. | Cucumis sativus, Arabidopsis thaliana | Protection against cucumber mosaic virus (CMV) via ISR. Higher number of leaves, increased fresh/dry weight, and the yield of cucumber. |
| | Allium cepa | Protection against white rot disease (Sclerotium cepivorum) with enhanced plant height, dry weight, and bulb perimeter. Enhancing levels of peroxidase and polyphenol oxidase. Upregulation of plant defense genes (PR1, PR2). |
| Phormidium sp. | Oryza sativa | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| Plectonema sp. | Oryza sativa | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| Purpureocillium sp. | Zea mays, Phaseolus vulgaris, Glycine max | Enhancing plant height and biomass and N content in roots (Z. mays) and P in shoots (P. vulgaris). P solubilization and IAA production. |
| Rhizobium sp. | Gossypium hirsutum | Increasing rate of seedling emergence, biomass, and N uptake and IAA synthesis. |
| | Oryza sativa | Increasing yield and uptake of N, P, K, and Fe. improved seed vigor, dry biomass, and leaf area with faster seedling emergence. IAA synthesis. |
| Rhodoferax sp | Zea mays, Beta vulgaris, Lactuca sativa, and Solanum lycopersicum | Growth the crops, microorganism can degrade chemical herbicides. |
| Scenedesmus sp. | Lycopersicon esculentum | Enhancing biomass and root length. |
| Serratia sp. | Solanum melongena | Salt stress alleviation, decreased Na+/Cl-content in leaves, lower lipid peroxidation level, and higher activity of antioxidant enzymes. Enhancing biomass, longer stems, and bigger leaf area. |
| | Triticum aestivum | Salt stress alleviation with enhanced plant height, root length, and yield, and higher chlorophyll content. ACC deaminase production. |
| Spirulina sp. | Zea mays | Cadmium stress alleviation with improved photosynthetic electron flows and increased non-photochemical quenching. Enhancing seed germination, shoot length, root fresh weight, and bigger leaf area. Decreasing Cd accumulation in shoot. |

(continued)

| Microorganism | Plant | Applications |
|---|---|---|
| *Streptomyces sp.* | *Arabidopsis thaliana, Lycopersicon esculentum* | Salt stress alleviation with increased biomass, chlorophyll content, and decreased proline content. Production of IAA, ACC deaminase, P, and NaCl solubilization. |
| | *Medicago sativa* | Protection against root-lesion nematode *(Pratylenchus penetrans)* |
| *Tolypothrix sp.* | *Oryza sativa* | Enhancing shoot and root length and biomass. Improving protein, phenolics, flavonoids, and chlorophyll content. Higher activity of enzymes (peroxidase, phenylalanine, and ammonia lyase). Elevating levels of IAA, and IBA. |
| *Trichoderma sp.* | *Allium cepa* | Protection against white rot disease *(Sclerotium cepivorum)* with enhanced plant height, dry weight, and bulb perimeter. Enhancing levels of peroxidase and polyphenol oxidase. Upregulation of plant defense genes *(PR1, PR2)*. |
| | *Freesia refracta* | Accelerating flowering and enhanced development of lateral inflorescence shoots. Increasing K, Fe, Mn, and Zn uptake. |
| | *Curcuma longa* | Enhancing plant height and yield. Biocontrol activity against rhizome rot and leaf blight *(Pythium aphanidermatum, Rhizoctonia solani)*. Production of IAA, HCN, cellulase, and P solubilization. |
| | *Cucumis melo* | Enhancing plant development, biomass, and fruit yield. Biocontrol activity against gummy stem blight pathogens *(Stagonosporopsis cucurbitacearum, Fusarium equiseti)*. |
| | *Brassica napus* | Enhancing biomass, lateral roots development, and germination ratio. Changes in microbial composition. |
| *Virgibacillus sp.* | *Oryza sativa, Solanum lycopersicum, Gossypium hirsutum and Zea mays* | Tolerance for abiotic stress and plant growth promoting. |

In some embodiments, the microbial composition containing a microorganism used in agriculture that is at least one specie selected from genus *Methylobacterium,* preferably the species *M. aquaticum* and *M. aminovorans.*

Another aspect of the invention is about the concentration of each strain contains in the microbial composition to be used such as in described in the present invention. Each strain in the microbial composition is at least $10^7$CFU/g by each microorganism thereof.

In the present invention, some embodiments of the microbial composition are applied to the plant formulated with compounds usually in the sector of agriculture. Following one example of a compound or a combination thereof selected from but not limited to the group consisting of adjuvants, alginic acid, amino acids, arabinogalactan, bioplastic, carbohydrates, carboxymethyl cellulose, carboxylic acids, cellulose, chelated minerals, chitosan, complex minerals, cyclodextrin, disaccharides, dispersants, fertilizer mixtures, fulvic acids, gum Arabic, humectant, humic acids, hydrogel, hydrolysates, hydrolyzed proteins, lignosulfonates, macronutrients, maltodextrins, maltodextrose, metal ions, methoxyl pectin, micronutrients, monosaccharides, organic acids, peptides, proteases, polyacrylic acid, salts, secondary-nutrients, silica, silver, sodium alginate, sodium selenosulfate, surfactants, titanium dioxide, UV protectants, vitamins, wetting agents, xantham gum and zinc oxide.

One embodiment of the present invention, the microbial application is preferably applied to phyllosphere by spraying. Phyllosphere or leaf surfaces have been shown to provide appropriate conditions for colonization by microorganisms including diazotrophic bacteria that are able to fix atmospheric nitrogen. The main microorganism contains in the microbial composition described in the present invention, *Bacillus infantis* deposited under number CECT 30759, is characterized by having capability of nitrogen fixation.

In another embodiment of the present invention, the microbial composition is applied as a solid formulation such as: Dustable powders; a free-flowing powder suitable for dusting, tablets for direct application; a formulation in the form of tablets to be applied individually and directly in the field and/or bodies of water, without preparation of a spraying solution or dispersion, emulsifiable granules; a granular formulation, which may contain water-insoluble formulants, to be applied as an oil-in-water emulsion of the active constituent(s) after disintegration in water, emulsifiable powders; a powder formulation, which may contain water-insoluble formulants, to be applied as an oil-in-water emulsion of the active constituent(s) after dispersion in water, granules; a free-flowing solid formulation of a defined granule size range ready to use, water-soluble gel; a gelatinized formulation to be applied as an aqueous solution, water-soluble granules; a formulation consisting of granules to be applied as a true solution of the active ingredient after dissolution in water, but which may contain insoluble inert ingredients, water-soluble powders; a powder formulation to be applied as a true solution of the active constituent after dissolution in water, but which may contain insoluble inert ingredients, water-soluble tablets; a formulation in the form of tablets to be used individually, to form a solution of the active ingredient after disintegration in water, water-dispersible granules; a formulation consisting of granules to be applied after disintegration and dispersion in water, wettable powders; a powder formulation to be applied as a suspension after dispersion in water, and water-dispersible tablets; a formulation in the form of tablets to be used individually, to form a dispersion of the active constituent after disintegration in water. In the present invention the microbial composition is applied preferably as powder in any variations previously described.

The microbial composition of the invention can be applied alone or in combination with additional components such as liquid fertilizer, amino acids and other products and techniques used in agriculture such as models e.g. embedded in computer programs for site specific crop management, satellite farming, precision farming or precision agriculture. Such models support the site-specific management of agricultural sites with data from various sources such as soils, weather, crops (e.g. type, growth stage, plant health), weeds (e.g. type, growth stage), diseases, pests, nutrients, water, moisture, biomass, satellite data, yield, etc. with the purpose to optimize profitability, sustainability

and protection of the environment. In particular, such models can help to optimize agronomical decisions, control the precision of pesticide applications and record the work performed.

The microbial composition can also be used in combination with smart spraying equipment such as e.g. spot spraying or precision spraying equipment attached to or housed within a farm vehicle such as a tractor, robot, helicopter, airplane, unmanned aerial vehicle (UAV) such as a drone, etc. Such an equipment usually includes input sensors (such as e.g. a camera) and a processing unit configured to analyze the input data and configured to provide a decision based on the analysis of the input data to apply the microbial composition of the invention to the crop plants (respectively the weeds) in a specific and precise manner. The use of such smart spraying equipment usually also requires positions systems (e.g. GPS receivers) to localize recorded data and to guide or to control farm vehicles; geographic information systems (GIS) to represent the information on intelligible maps, and appropriate farm vehicles to perform the required farm action such as the spraying. In an example, plant pests can be detected from imagery acquired by a camera. In an example, plant pests can be identified and/or classified based on that imagery. Such identification and/ classification can make use of image processing algorithms. Such image processing algorithms can utilize machine learning algorithms, such as trained neutral networks, decision trees and utilize artificial intelligence algorithms. In this manner, the microbial composition described herein can be applied only where needed. If not mentioned otherwise the treatment of plants or plant parts (which includes seeds and plants emerging from the seed), harvested fruits and vegetables with the microbial composition according to the invention is carried out directly or by action on their surroundings, habitat or storage space using customary treatment methods, for example dipping, spraying, atomizing, irrigating, evaporating, dusting, fogging, broadcasting, foaming, painting, spreading-on, watering (drenching), drip irrigating.

In another aspect, the present invention relates to a method for improving mineral nutrition and promoting the growth in crop yield and/or the quality of food commodities comprising the step of applying the microbial composition as defined to a plant, plant part or seed or a locus where said plant or seed is intended to be grown. Plants and plant parts. The microbial composition of the invention may be applied to any plants or plant parts, preferably in the phyllosphere.

Plants mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the genetically modified plants (GMO or transgenic plants) and the plant cultivars which are protectable and non-protectable by plant breeder's rights. Plant cultivars are understood to mean plants which have new properties ("traits") and have been obtained by conventional breeding, by mutagenesis or by recombinant DNA techniques. They can be cultivars, varieties, bio- or genotypes. Plant parts are understood to mean all parts and organs of plants above and below the ground, such as shoots, leaves, needles, stalks, stems, flowers, fruit bodies, fruits, seeds, roots, tubers and rhizomes. The plant parts also include harvested material and vegetative and generative propagation material, for example cuttings, tubers, rhizomes, slips and seeds. Plants which may be treated in accordance with the methods of the invention include the following: cotton, flax, grapevine, fruit, vegetables, such as Rosaceae sp. (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae sp.* (for example cucumber), Alliaceae sp. (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae* sp. (for example white cabbage, red cabbage, broccoli, cauliflower, Brussels sprouts, pak choi, kohlrabi, radishes, and oilseed rape, mustard, horseradish and cress), *Fabacae* sp. (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants. Plants and plant cultivars which may be treated by the above disclosed methods include plants and plant cultivars which are resistant against one or more biotic stresses, i.e. said plants show a better defense against animal and microbial pests, such as against nematodes, insects, mites, phytopathogenic fungi, bacteria, viruses and/or viroids. Plants and plant cultivars which may be treated by the above disclosed methods include those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance. Plants and plant cultivars which may be treated by the above disclosed methods include those plants characterized by enhanced yield characteristics. Increased yield in said plants may be the result of, for example, improved plant physiology, growth and development, such as water use efficiency, water retention efficiency, improved nitrogen use, enhanced carbon assimilation, improved photosynthesis, increased germination efficiency and accelerated maturation. Yield may furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content and composition for example cotton or starch, protein content, oil content and composition, nutritional value, reduction in antinutritional compounds, improved processability and better storage stability. Plants and plant cultivars which may be treated by the above disclosed methods include plants and plant cultivars which are hybrid plants that already express the characteristic of heterosis or hybrid vigor which results in generally higher yield, vigor, health and resistance towards biotic and abiotic stresses. Transgenic plants and integration events.

The microbial composition according to the invention can be advantageously used to treat transgenic plants, plant cultivars or plant parts that received genetic material which imparts advantageous and/or useful properties (traits) to these plants, plant cultivars or plant parts. Therefore, it is contemplated that the present invention may be combined with one or more recombinant traits or transgenic event(s) or a combination thereof. For the purposes of this application, a transgenic event is created by the insertion of a specific recombinant DNA molecule into a specific position (locus) within the chromosome of the plant genome. The insertion creates a novel DNA sequence referred to as an "event" and is characterized by the inserted recombinant DNA molecule and some amount of genomic DNA immediately adjacent to/flanking both ends of the inserted DNA. Such trait(s) or transgenic event(s) include, but are not limited to, pest resistance, water use efficiency, yield performance, drought tolerance, seed quality, improved nutritional quality, hybrid seed production, and herbicide tolerance, in which the trait is measured with respect to a plant lacking such trait or transgenic event. Concrete examples of such advantageous and/or useful properties (traits) are better plant growth, vigor, stress tolerance, standability, lodging resistance, nutrient uptake, plant nutrition, and/or yield, in particular improved growth, increased tolerance to high or low temperatures, increased tolerance to drought or to levels of water or soil salinity, enhanced flowering performance, easier harvesting, accelerated ripening, higher yields, higher quality and/or a higher nutritional value of the harvested products, better storage life and/or processability of the harvested products, and increased resistance against animal and microbial pests, such as against insects, arachnids, nematodes, mites, slugs and snails. Among DNA sequences encoding proteins which confer properties of tolerance to such animal and microbial pests, in particular insects, mention will particularly be made of the genetic material from *Bacillus thuringiensis* encoding the Bt proteins widely described in the literature and well known to those skilled in the art. Mention will also be made of proteins extracted from bacteria such as Photorhabdus (WO97/17432 and WO98/08932). In particular, mention will be made of the Bt Cry or VIP proteins which include the CrylA, CrylAb, CrylAc, CryIIA, CrylIIA, CrylIIB2, Cry9c Cry2Ab, Cry3Bb and CrylF proteins or toxic fragments thereof and also hybrids or combinations thereof, especially the CrylF protein or hybrids derived from a CrylF protein (e.g. hybrid CrylACrylF proteins or toxic fragments thereof), the CrylA-type proteins or toxic fragments thereof, preferably the CrylAc protein or hybrids derived from the CrylAc protein (e.g. hybrid CrylAb-CrylAc proteins) or the CrylAb or Bt2 protein or toxic fragments thereof, the Cry2Ae, Cry2Af or Cry2Ag proteins or toxic fragments thereof, the CrylA. 105 protein or a toxic fragment thereof, the VIP3Aa19 protein, the VIP3Aa20 protein, the VIP3A proteins produced in the COT202 or COT203 cotton events, the VIP3Aa protein or a toxic fragment thereof as described in Estruch et al.

(1996), Proc Natl Acad Sci US A. 28;93(11):5389-94, the Cry proteins as described in WO2001/47952, the insecticidal proteins from *Xenorhabdus* (as described in WO98/50427), *Serratia* (particularly from S. entomophila) or *Photorhabdus* species strains, such as Tc-proteins from *Photorhabdus* as described in WO98/08932. Also, any variants or mutants of any one of these proteins differing in some amino acids (1-10, preferably 1-5) from any of the above-named sequences, particularly the sequence of their toxic fragment, or which are fused to a transit peptide, such as a plastid transit peptide, or another protein or peptide, is included herein. Another and particularly emphasized example of such properties is conferred tolerance to one or more herbicides, for example imidazolinones, sulphonylureas, glyphosate or phosphinothricin. Among DNA sequences encoding proteins which confer properties of tolerance to certain herbicides on the transformed plant cells and plants, mention will be particularly be made to the bar or PAT gene or the Streptomyces coelicolor gene described in WO2009/152359 which confers tolerance to glufosinate herbicides, a gene encoding a suitable EPSPS (5-Enolpyruvylshikimat-3-phosphat-synthase) which confers tolerance to herbicides having EPSPS as a target, especially herbicides such as glyphosate and its salts, a gene encoding glyphosate-n-acetyltransferase, or a gene encoding glyphosate oxidoreductase. Further suitable herbicide tolerance traits include at least one ALS (acetolactate synthase) inhibitor (e.g. WO2007/024782), a mutated Arabidopsis ALS/AHAS gene (e.g. U.S. Patent 6,855,533), genes encoding 2,4-D monooxygenases conferring tolerance to 2,4-D (2,4- dichlorophenoxyacetic acid) and genes encoding Dicamba monooxygenases conferring tolerance to dicamba (3,6- 10 dichloro-2-methoxybenzoic acid). Yet another example of such properties is resistance to one or more phytopathogenic fungi, for example Asian Soybean Rust. Among DNA sequences encoding proteins which confer properties of resistance to such diseases, mention will particularly be made of the genetic material from *Glycine tomentella,* for example from any one of available accession lines PI441001, PI483224, PI583970, PI446958, PI499939, PI505220, PI499933, PI441008, PI505256 or PI446961 as described in WO2019/103918. Further and particularly emphasized examples of such properties are increased resistance against bacteria and/or viruses owing, for example, to systemic acquired resistance (SAR), systemin, phytoalexins, elicitors and also resistance genes and correspondingly expressed proteins and toxins. Particularly useful transgenic events in transgenic plants or plant cultivars which can be treated with preference in accordance with the invention include Event 531/ PV-GHBK04 (cotton, insect control, described in WO2002/040677), Event 1143-14A (cotton, insect control, not deposited, described in WO2006/128569); Event 1143-51B (cotton, insect control, not deposited, described in WO2006/128570); Event 1445 (cotton, herbicide tolerance, not deposited, described in US-A 2002-120964 or WO2002/034946); Event 17053 (rice, herbicide tolerance, deposited as PTA-9843, described in WO2010/117737); Event 17314 (rice, herbicide tolerance, deposited as PTA-9844, described in WO2010/117735); Event 281-24-236 (cotton, insect control - herbicide tolerance, deposited as PTA-6233, described in WO2005/103266 or US-A 2005-216969); Event 3006-210-23 (cotton, insect control - herbicide tolerance, deposited as PTA-6233, described in US-A 2007-143876 or WO2005/103266); Event 3272 (corn, quality trait, deposited as PTA-9972, described in WO2006/098952 or US-A 2006- 35 230473); Event 33391 (wheat, herbicide tolerance, deposited as PTA-2347, described in WO2002/027004), Event 40416 (corn, insect control - herbicide tolerance, deposited as ATCC PTA-11508, described in WO 11/075593); Event 43A47 (corn, insect control - herbicide tolerance, deposited as ATCC PTA-11509, described in WO2011/075595); Event 5307 (corn, insect control, deposited as ATCC PTA-9561, described in WO2010/077816); Event ASR-368 (bent grass, herbicide tolerance, deposited as ATCC PTA-4816, described in US-A 2006-162007 or WO2004/053062); Event B16 (corn, herbicide tolerance, not deposited, described in US-A 2003-126634); Event BPS-CV127-9 (soybean, herbicide tolerance, deposited as NCIMB No. 41603, described in WO2010/080829); Event BLRI (oilseed rape, restoration of male sterility, deposited as NCIMB 41193, described in WO2005/074671), Event CE43-67B (cotton, insect control, deposited as DSM ACC2724, described in US-A 2009-217423 or WO2006/128573); Event CE44-69D (cotton, insect control, not deposited, described in US-A 2010- 0024077); Event CE44-69D (cotton, insect control, not deposited, described in WO2006/128571); Event CE46-02A (cotton, insect control, not deposited, described in 15 WO2006/128572); Event COT102 (cotton, insect control, not deposited, described in US-A 2006-130175 or WO2004/039986); Event COT202 (cotton, insect control, not deposited, described in US-A 2007-067868 or WO2005/054479); Event COT203 (cotton, insect control, not deposited, described in WO2005/054480); ); Event DAS21606-3/1606 (soybean, herbicide tolerance, deposited as PTA-11028, described in WO2012/033794), Event DAS40278 (corn, herbicide tolerance, deposited as ATCC PTA-10244, described in WO2011/022469); Event DAS-44406-6/ pDAB8264.44.06.1 (soybean, herbicide tolerance, deposited as PTA-11336, described in WO2012/075426), Event DAS-14536-7/pDAB8291.45.36.2 (soybean, herbicide tolerance, deposited as PTA-11335, described in WO2012/075429), Event DAS-59122-7 (corn, insect control - herbicide tolerance, deposited as ATCC PTA 11384, described in US-A 2006-070139); Event DAS-59132 (corn, insect control - herbicide tolerance, not deposited, described in WO2009/100188); Event DAS68416 (soybean, herbicide tolerance, deposited as ATCC PTA-10442, described in WO2011/066384 or WO2011/066360); Event DP-098140-6 (corn, herbicide tolerance, deposited as ATCC PTA-8296, described in US-A 2009- 30 137395 or WO 08/112019); Event DP-305423-1 (soybean, quality trait, not deposited, described in US-A 2008-312082 or WO2008/054747); Event DP-32138-1 (corn, hybridization system, deposited as ATCC PTA-9158, described in US-A 2009-0210970 or WO2009/103049); Event DP-356043-5 (soybean, herbicide tolerance, deposited as ATCC PTA-8287, described in US-A 2010-0184079 or WO2008/002872); Event EE-I (brinjal, insect control, not deposited, described in WO 07/091277); Event Fil 17 (corn, herbicide tolerance, deposited as ATCC 209031, described in US-A 2006-059581 or WO 98/044140); Event FG72 (soybean, herbicide tolerance, deposited as PTA-11041, 31 described in WO2011/063413), Event GA21 (corn, herbicide tolerance, deposited as ATCC 209033, described in US-A 2005-086719 or WO 98/044140); Event GG25 (corn, herbicide tolerance, deposited as ATCC 209032, described in US-A 2005-188434 or WO98/044140); Event GHB119 (cotton, insect control, herbicide tolerance, deposited as ATCC PTA-8398, described in WO2008/151780); Event GHB614 (cotton, herbicide tolerance, deposited as ATCC PTA-6878, described in US-A 2010-050282 or WO2007/017186); Event GJ11 (corn, herbicide tolerance, deposited as ATCC 209030, described in US-A 2005-188434 or WO98/044140); Event GM RZ13 (sugar beet, virus resistance, deposited as NCIMB-41601, described in WO2010/076212); Event H7-I (sugar beet, herbicide tolerance, deposited as NCIMB 41158 or NCIMB 41159, described in US-A 2004-172669 or WO 2004/074492); Event JOPLINI (wheat, disease tolerance, not deposited, described in US-A 2008-064032); Event LL27 (soybean, herbicide tolerance, deposited as NCIMB41658, described in WO2006/108674 or US-A 2008-320616); Event LL55 (soybean, herbicide tolerance, deposited as NCIMB 41660, described in WO 2006/108675 or US-A 2008-196127); Event LLcotton25 (cotton, herbicide tolerance, deposited as ATCC PTA-3343, described in WO2003/013224 or US- A 2003-097687); Event LLRICE06 (rice, herbicide tolerance, deposited as ATCC 203353, described in US 6,468,747 or WO2000/026345); Event LLRice62 ( rice, herbicide tolerance, deposited as ATCC 203352, described in WO2000/026345), Event LLRICE601 (rice, herbicide tolerance, deposited as ATCC PTA-2600, described in USA 2008-2289060 or WO2000/026356); Event LY038 (corn, quality trait, deposited as ATCC PTA-5623, described in US-A 2007-028322 or WO2005/061720); Event MIR162 (corn, insect control, deposited as PTA-8166, described in US-A 2009-300784 or WO2007/142840); Event MIR604 (corn, insect control, not deposited, described in US25 A 2008-167456 or WO2005/103301); Event MON15985 (cotton, insect control, deposited as ATCC PTA-2516, described in US-A 2004-250317 or WO2002/100163); Event MON810 (corn, insect control, not deposited, described in US-A 2002-102582); Event MON863 (corn, insect control, deposited as ATCC PTA-2605, described in WO2004/011601 or US-A 2006-095986); Event MON87427 (corn, pollination control, deposited as ATCC PTA-7899, described in WO2011/062904); Event MON87460 (corn, stress tolerance, deposited as ATCC PTA-8910, described in WO2009/111263 or US-A 2011-0138504); Event MON87701 (soybean, insect control, deposited as ATCC PTA 8194, described in US-A 2009-130071 or WO2009/064652); Event MON87705 (soybean, quality trait - herbicide tolerance, deposited as ATCC PTA-9241, described in 35 US-A 2010-0080887 or WO2010/037016); Event MON87708 (soybean, herbicide tolerance, deposited as ATCC PTA-9670, described in WO2011/034704); Event MON87712 (soybean, yield, deposited as PTA-10296, described in WO2012/051199), Event MON87754 (soybean, quality trait, deposited as ATCC PTA-9385, described in WO2010/024976); Event MON87769 (soybean, quality trait, deposited as ATCC PTA8911, described in US-A 2011-0067141 or WO2009/102873); Event MON88017 (corn, insect control - herbicide tolerance, deposited as ATCC PTA-5582, described in US-A 2008-028482 or WO2005/059103); Event MON88913 (cotton, herbicide tolerance, deposited as ATCC PTA-4854, described in WO2004/072235 or US-A 2006-059590); Event MON88302 (oilseed rape, herbicide tolerance, deposited as PTA-10955, described in WO2011/153186), Event MON88701 (cotton, herbicide tolerance, deposited as PTA-11754, described in WO2012/134808), Event MON89034 (corn, insect control, deposited as ATCC PTA-7455, described in WO 07/140256 or US-A 2008-260932); Event MON89788 (soybean, herbicide tolerance, deposited as ATCC PTA-6708, described in US-A 2006-282915 or WO2006/130436); Event MSI 1 (oilseed rape, pollination control - herbicide tolerance, deposited as ATCC PTA-850 or PTA-2485, described in WO2001/031042); Event MS8 (oilseed rape, pollination control - herbicide tolerance, deposited as ATCC PTA-730, described in WO2001/041558 or US-A 2003-188347); Event NK603 (corn, herbicide tolerance, deposited as ATCC PTA-2478, described in US-A 2007-292854); Event PE-7 (rice, insect control, not deposited, described in WO2008/114282); Event RF3 (oilseed rape, pollination control - herbicide tolerance, deposited as ATCC PTA-730, described in WO2001/041558 or US-A 2003-20 188347); Event RT73 (oilseed rape, herbicide tolerance, not deposited, described in WO2002/036831 or US-A 2008-070260); Event SYHT0H2 / SYN-000H2-5 (soybean, herbicide tolerance, deposited as PTA-11226, described in WO2012/082548), Event T227-1 (sugar beet, herbicide tolerance, not deposited, described in WO2002/44407 or US-A 2009-265817); Event T25 (corn, herbicide tolerance, not deposited, described in US-A 2001-029014 or WO2001/051654); Event T304-40 (cotton, insect control - herbicide tolerance, deposited as ATCC PTA-8171, described in US-A 2010-077501 or WO2008/122406); Event T342-142 (cotton, insect control, not deposited, described in WO2006/128568); Event TC1507 (corn, insect control - herbicide tolerance, not deposited, described in US-A 2005-039226 or WO2004/099447); Event VIP1034 (corn, insect control - herbicide tolerance, deposited as ATCC PTA-3925, described in WO2003/052073), Event 32316 (corn, insect control-herbicide tolerance, deposited as PTA-11507, described in WO2011/084632), Event 4114 (corn, insect control-herbicide tolerance, deposited as PTA-11506, described in WO2011/084621), Event EE-GME/ FG72 (soybean, herbicide tolerance, ATCC Accession N° PTA-11041) optionally stacked with Event EE-GM1/LL27 or Event EE-GM2/LL55 (WO2011/063413A2), Event DAS-68416-4 (soybean, herbicide tolerance, ATCC Accession N° PTA-10442, WO2011/066360AI), Event DAS-68416-4 (soybean, herbicide tolerance, ATCC Accession N° PTA-10442, WO2011/066384AI), Event DP-040416-8 (corn, insect control, ATCC Accession N° PTA-11508, WO2011/075593AI), Event DP-043A47-3 (corn, insect control, ATCC Accession N° PTA-11509, WO2011/075595AI), Event DP-004114-3 (corn, insect control, ATCC Accession N° PTA-11506, WO2011/084621AI), event DP 032316-8 (corn, insect control, ATCC Accession N° PTA-11507, WO2011/084632AI), Event MON-88302-9 (oilseed rape, herbicide tolerance, ATCC Accession N° PTA-10955, WO2011/153186AI), Event DAS-21606-3 (soybean, herbicide tolerance, ATCC Accession No. PTA-11028, WO2012/033794A2), Event MON-87712-4 (soybean, quality trait, ATCC Accession N°. PTA-10296, WO2012/051199A2), Event DAS-44406-6 10 (soybean, stacked herbicide tolerance, ATCC Accession N°. PTA-11336, WO2012/075426AI), Event DAS-14536-7 (soybean, stacked herbicide tolerance, ATCC Accession N°. PTA-11335, WO2012/075429AI), Event SYN-000H2-5 (soybean, herbicide tolerance, ATCC Accession N°. PTA-11226, WO2012/082548A2), Event DP 061061-7 (oilseed rape, herbicide tolerance, no deposit N° available, WO2012071039AI), Event DP-073496-4 (oilseed rape, herbicide tolerance, no deposit N° available, US2012131692), Event 8264.44.06.1 (soybean, stacked herbicide tolerance, Accession N° PTA-11336, WO2012075426A2), Event 8291.45.36.2 (soybean, stacked herbicide tolerance, Accession N°. PTA-11335, WO2012075429A2), Event SYHT0H2 (soybean, ATCC Accession N°. PTA-11226, WO2012/082548A2), 20 Event MON88701 (cotton, ATCC Accession N° PTA-11754, WO2012/134808AI), Event KK179-2 (alfalfa, ATCC Accession N° PTA-11833, WO2013/003558AI), Event pDAB8264.42.32.1 (soybean, stacked herbicide tolerance, ATCC Accession N° PTA 11993, WO2013/010094AI), Event MZDT09Y (corn, ATCC Accession N° PTA-13025, WO2013/012775AI). Further, a list of such transgenic event(s) is provided by the United States Department of Agriculture's (USDA) Animal and Plant Health Inspection Service (APHIS) and can be found on their website on the world wide web at aphis.usda.gov. For this application, the status of such list as it is/was on the filing date of this application, is relevant. The genes/events which impart the desired traits in question may also be present in combinations with one another in the transgenic plants. Examples of transgenic plants which may be mentioned are the important crop plants, such as cereals (wheat, rice, triticale, barley, rye, oats), corn, soya beans, potatoes, sugar beet, sugar cane, tomatoes, peas and other types of vegetable, cotton, tobacco, oilseed rape and also fruit plants (with the fruits apples, pears, citrus fruits and grapes), with particular emphasis being given to corn, soya beans, wheat, rice, potatoes, cotton, sugar cane, tobacco and oilseed rape.

## Brief description of drawings

**Figure 1:** Petri dishes cultured with *Bacillus infantis* CECT 30759 (c), positive control (a), and negative control (b) for amylase activity.

**Figure 2** shows an amylase activity diagram of the size for colonies (1) and clearance (2) from *Bacillus infantis* CECT 30759 (MG) compared to positive (MS1) and negative control (C-).

**Figure 3** shows petri dish for culture medium described with *Bacilllus infantis* CECT 30759 for protease activity.

**Figure 4** represents a protease activity diagram of the size for colonies (1) and clearance (2) MG (*Bacilllus infantis* CECT 30759), MS1 (positive control) C- (negative control, water).

**Figure 5** represents a cellulose activity diagram of the size for colonies (1) and clearance (2). MG (*Bacilllus infantis* CECT 30759), MS1 (positive control) and C- (negative control, water).

**Figure 6** shows petri dishes in culture medium described: (a) MS1 (positive control) and C- (negative control), (b) MG (*Bacilllus infantis* CECT 30759) for cellulose activity.

**Figure 7** represents a diagram of measurement of absorbance for negative control (C-), biosurfactant producing microorganism (EMC3) and *Bacilllus infantis* CECT 30759 (MG).

**Figure 8** represents production of ethylene due to the nitrogenase activity from *Bacilllus infantis* CECT 30759.

**Figure 9** represents a diagram of mean value for height; *Bacilllus infantis* CECT 30759 (MG) commercial product (CP) or organic fertilizer (CN 70%).

**Figure 10** represents a diagram of mean value for fresh weight; *Bacilllus infantis* CECT 30759 (MG) commercial product (CP) or organic fertilizer (CN 70%).

**Figure 11** represents a diagram of mean value for dry weight; *Bacilllus infantis* CECT 30759 (MG) commercial product (CP) or organic fertilizer (CN 70%).

**Figure 12** represents a diagram of the manufacturing process of the microbial composition: (1) starter, (2) bioreactor, (3) centrifugal separator, (4) formulation, (5) dryer and (6) bacterial powder

**Figure 13** represents a diagram of mean value for height; Microbial composition (MC) commercial product (CP), organic fertilizer containing 70% nitrogen (CN 70%) and organic fertilizer 100% nitrogen (CN 100%).

**Figure 14** represents a diagram of mean value for fresh weight; Microbial composition (MC) commercial product (CP), organic fertilizer containing 70% nitrogen (CN 70%) and organic fertilizer 100% nitrogen (CN 100%).

**Figure 15** represents a diagram of mean value for % fresh weight/dry weight: Microbial composition (MC) commercial product (CP), organic fertilizer containing 70% nitrogen (CN 70%) and organic fertilizer 100% nitrogen (CN 100%).

## Description of embodiments

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art.

The singular form "a", "an", and "the" include plural references unless the context clearly dictates otherwise.

ACC means aminocyclopropane-1-carboxylic acid.

Adjuvants is defined as a supplemental substance added to a mixture to enhance the performance and/or physical properties of the desired chemical.

Biostimulant is defined as any substance or microorganism applied to plants with the aim to enhance nutrition efficiency, abiotic stress tolerance and/or crop quality traits, regardless of its nutrients content. By extension, plant biostimulants also designate commercial products containing mixtures of such substances and/or microorganisms. Furthermore, the proposed definition aims at contributing to the acceptance of biostimulants by future regulations, especially in the EU, drawing the lines between biostimulants and fertilisers, pesticides or biocontrol agents. In this sense, microbial biostimulants include mycorrhizal and non-mycorrhizal fungi, bacterial endosymbionts and Plant Growth-Promoting Bacteria. Thus, microorganisms applied to plants can have a dual function of biocontrol agent and of biostimulant, and the claimed agricultural effect will be instrumental in their regulatory categorization. (Jardin 2015).

Biosurfactants usually refers to surfactants of microbial origin, herein are understood as substances produced by microbes that are synthesized extracellularly. As a secondary metabolite of microorganisms, biosurfactants can be processed by the cultivation of biosurfactant producing microorganisms in the stationary phase on many sorts of low-priced substrates like biochar, plant oils, carbohydrates, wastes, etc.

Colony-forming unit (CFU) is a unit which estimates the number of microbial cells (bacteria, fungi, viruses etc.) in a sample that are viable, able to multiply via binary fission under the controlled conditions.

Composition: as used herein "composition" is defined as a combination of active ingredients (microorganisms) and another compound, carrier, composition, or element inert (for example: surfactant or wetting).

Dose or effective amount, as used herein, is an amount sufficient to affect desired results. An effective amount can be administered in one or more applications.

GA means gibberellins or gibberellic acid.

IAA means indole-3-acetic acid.

IBA means Indole-3-butyric acid.

JA means jasmonic acid.

*Methylobacterium sp.* or *Methylorubrum sp.* must be considered the same genus in the present invention.

Microorganism or microbe is an organism of microscopic size, which may exist in its single-celled form or as a colony of cells. Microorganisms include most unicellular organisms from all three domains of life they can be extremely diverse. Two of the three domains, Archaea and Bacteria, only contain microorganisms. The third domain Eukaryote includes all multicellular organisms as well as many unicellular protists and protozoans that are microbes. There are also many multicellular organisms that are microscopic, namely micro-animals, some fungi, and some algae, but these are generally not considered microorganisms. In the present invention can be referred mainly to Bacteria but may include another organism as previously defined.

Plant growth promoting bacteria or PGPB, as used herein, means bacteria that can enhance plant growth and protect plants from disease and abiotic stresses through a wide variety of mechanisms; those, that establish close associations with plants, could be more successful in plant growth promotion. Several important bacterial characteristics, such as biological nitrogen fixation, potassium and phosphate solubilization, enzymological activity, and production of siderophores and phytohormones can be assessed as plant growth promotion (PGP) traits.

SA means salicylic acid.

Strain defined as a subset of a bacterial species differing from other bacteria of the same species by some minor, but identifiable difference.

Finally, it is also noted that the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

**Example 1: *Bacillus infantis* CECT 30759 as a plant growth promoting bacteria (PGPB).**

*Bacilllus infantis* CECT 30759 was tested in order to prove that can be defined as PGPB. In the present invention, the mechanisms by which PGPB stimulate plant growth involve the availability of nutrients originating from genetic processes such as biological nitrogen fixation; different enzymological activity (amylase, cellulase, and protease); and production of phytohormones.

Amylase activity from *Bacillus infantis* CECT 30759.

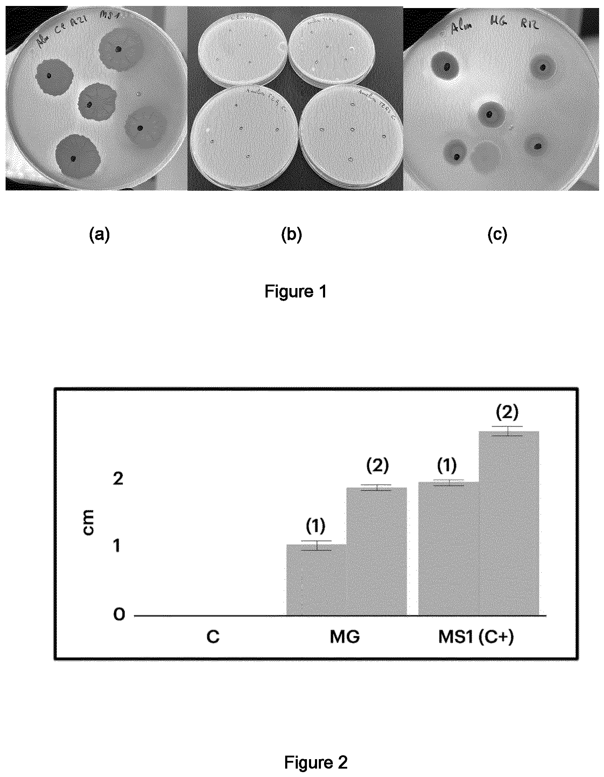

*Bacilllus infantis* CECT 30759 was tested to determine, if it is capable of breaking down starch into maltose through the activity of the extra-cellular $\alpha$-amylase enzyme. Use a sterile loop to pick a few colonies from the pure culture plate to petri dish with medium culture of agar enriched in starch. To Incubate plate at 37 °C for 72 hours and to add 2-3 drops of 10% iodine solution directly onto the edge of colonies. After 10- 15 minutes, the areas surrounding isolated colonies where starch has been hydrolyzed by amylase will appear clear shown in the **Figure 1. Figure 2** shows the amylase activity from *Bacillus infantis* CECT 30759.

Protease activity from *Bacillus infantis* CECT 30759

*Bacilllus infantis* CECT 30759 was tested on petri dish that allow to evaluate its protease activity.

Proteases belong to hydrolase class of enzymes which are specific for the hydrolysis of the peptide bonds present in the polypeptide chain of amino acids to produce free amino acids and peptides. Proteases are thus those enzymes which are degradative in nature and shows great specificity and selectivity during protein modifications.

Proteases form an integral component of life on earth, and are ubiquitously found in plants, animals, and several species of microbes such as bacteria, fungi, and yeast. Genus *Bacillus sp.* is the most active and dynamic extracellular alkaline protease producers in the industrial sector and thus constitute the major source of commercial proteases.

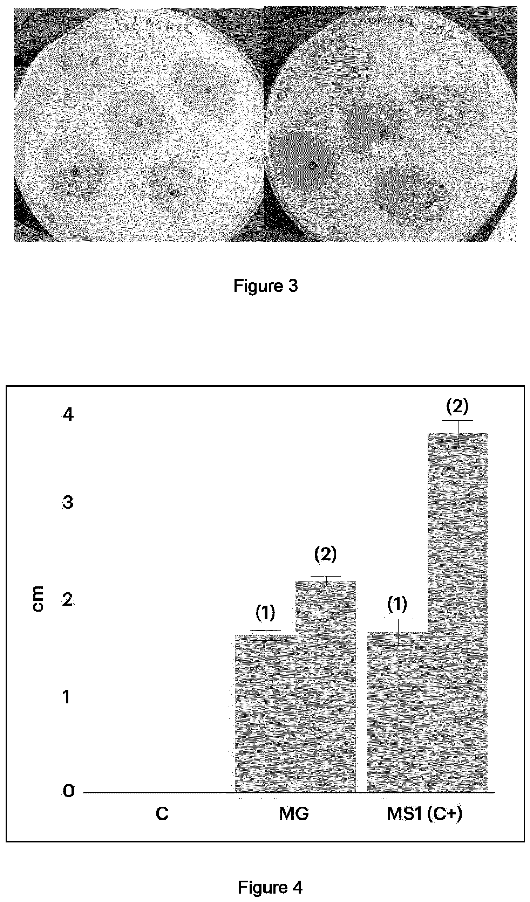

*Bacilllus infantis* CECT 30759 strain was investigated for its protease enzyme activity on skimmed-milk medium that was prepared using agar 15 g/L, casein peptone 5 g/L, skim milk powder 20 g/L and yeast-extract 0.25 g/L, having basic pH. The media was autoclaved for 10 min at 121 °C, cooled, dispensed into plates and solidified. After inoculating a loop-full of inoculum for every bacterial isolate on the prepared plates, they were placed in incubation at 37°C for 48 hours. (Figure 3 & 4). On completion of the incubation period, the zone of hydrolytic clearance surrounding the colonies of each isolate depicting protease production were checked and the colony for *Bacilllus infantis* CECT 30759 showed hydrolytic zone.

Cellulose activity from *Bacilllus infantis* CECT 30759

*Bacilllus infantis* CECT 30759 was tested on petri dish that allow to demonstrate its cellulase activity.

Cellulose is the most abundant biomass on Earth. It is the primary product of photosynthesis in terrestrial environments and the most abundant renewable bioresource produced in the biosphere. Cellulose is commonly degraded by an enzyme called cellulase. This enzyme is produced by several microorganisms, commonly by bacteria and fungi. Cellulase yields appear to depend upon a complex relationship involving a variety of factors like inoculums size, pH value, temperature, presence of inducers, medium additives, aeration, growth time, and so forth.

*Bacilllus infantis* CECT 30759 was cultured along with a positive control (MS1) and negative control (water) in the following medium contained cellulose 2 g/L, gelatin 2 g/L, agar 15 g/L, $KH_2PO_4$ 0.5 g/L, and $MgSO_4$ 0.25 g/L and Congo red 15 ml/L. First dissolve the cellulose applying heat and agitation, until the cellulose is not completely dissolved the rest of the components of the medium should not be added. Once the medium comes out of the autoclave, homogenize again by stirring. Finally let it cool down to 50-55°C and add Congo Red, adjusted pH to the range 6.8-7.2. The inoculated medium was incubated at 37°C in shaker incubator for 7 days.

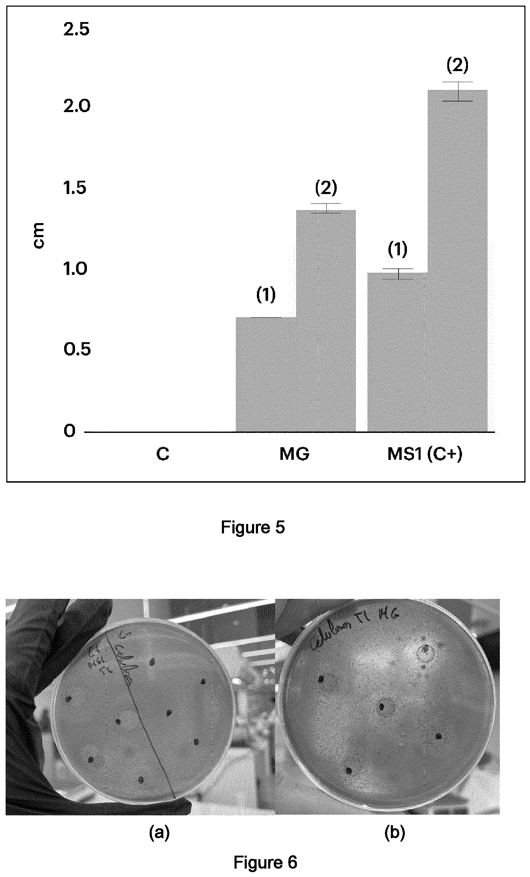

If the strain has cellulose activity, then a zone of hydrolytic clearance surrounding the colonies will be found. The colony for *Bacilllus infantis* CECT 30759 showed it. **Figure 5 and 6.**

Production of phytohormones from *Bacilllus infantis* CECT 30759

In the present invention, *Bacilllus infantis* CECT 30759 was tested to evaluate the production of phytohormones. *Bacilllus infantis* CECT 30759 was inoculated in erlenmeyer flaskwith lysogeny broth (LB) liquid culture for 48 hours at 28 °C and 160rpm.

Posteriorly, microbial growth was centrifuged for 15 minutes to 6000 rpm, the culture supernatant is analyzed to identify phytohormones and the pellet was assessed to detect potential contaminants.

The method to identify and quantify phytohormones was chromatography coupled with mass spectrometry (Agilent 6420). The result was that *Bacilllus infantis* CECT 30759 produces auxins, salic acid, and isopentyl adenine.

## Example 2: *Bacillus infantis* CECT 30759 as a biosurfactant

One example of the present invention shows *Bacilllus infantis* CECT 30759 as a biosurfactant. Biosurfactants are structurally a very diverse group of biomolecules, e.g., glycolipids, lipopeptides, lipoproteins, lipopolysaccharides or phospholipids. Therefore, most methods for a general screening of biosurfactant producing strains are based on the physical effects of surfactants.

For the growth of biosurfactant producing microbes, enrichment cultures utilizing hydrophobic compounds as the sole carbon source are applied. In this case, a glycerol medium was used to evaluate whether the external biomolecules produced by *Bacilllus infantis* CECT 30759 are tensioactive agents, and therefore biosurfactants.

Two methods were used to evaluate the production of biosurfactants by *Bacilllus infantis* CECT 30759:

- Drop collapse assay; This assay relies on the destabilization of liquid droplets by surfactants. Therefore, drops of a cell suspension or of culture supernatant are placed on an oil coated, solid surface. If the liquid does not contain surfactants, the polar water molecules are repelled from the hydrophobic surface and the drops remain stable. If the liquid contains surfactants, the drops spread or even collapse because the force or interfacial tension between the liquid drop and the hydrophobic surface is reduced. The stability of drops is dependent on surfactant concentration and correlates with surface and interfacial tension.

- Microplate assay; The surface activity of individual strains can be determined qualitatively with the microplate assay. This assay is based on the change in optical distortion that is caused by surfactants in an aqueous solution. Pure water

in a hydrophobic well has a flat surface. The presence of surfactants causes some wetting at the edge of the well and the fluid surface becomes concave and takes the shape of a diverging lens. For this assay, a 100 $\mu$l sample of the supernatant of each strain is taken and put into a microwell of a 96-mircowell plate. The plate is viewed using a backing sheet of paper with a grid. If biosurfactant is present, the concave surface distorts the image of the grid below. The optical distortion of the grid provides a qualitative assay for the presence of surfactants.

*Bacilllus infantis* CECT 30759 was tested along with a positive control (Biosurfactant producing microorganisms) and negative control (water). As a result, *Bacilllus infantis* CECT 30759 had been able to grow in a medium for biosurfactant microorganisms.

**(Figure 7)**

## Example 3: *Bacillus infantis* as a fixing nitrogen microorganism

One example of present invention *Bacilllus infantis* CECT 30759 was tested for measuring nitrogen fixation rate through acetylene reduction assay (ARA), which relies on the preferential reduction of acetylene ($C_2H_2$) to ethylene ($C_2H_4$) by nitrogenase.

*Bacilllus infantis* CECT 30759 was inoculated in assay tube with Burk medium (No nitrogen and glucose as carbon source). After several days is added acetylene up to 10% of medium. As detection method is used the flame Ionization detector (FID) in Gas Chromatography (GC) for identifying and measuring ethylene for long-time period. *Azotobacter chroococcum* is used as positive control.

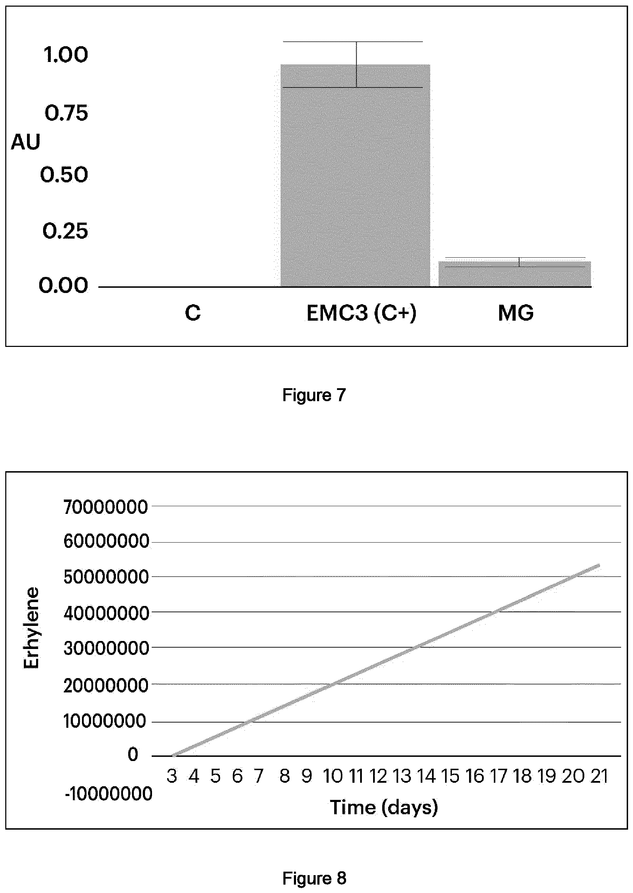

*Bacilllus infantis* CECT 30759 was inoculated for initial low optical density (OD 600nm=0.2 - 0.3), After 21 days for incubation at 28°C, the production of ethylene was measured at day 3 and 21 from the addition of ethylene. **(Figure 8)**

As a result, acetylene reduction assay (ARA) demonstrated the capacity of *Bacilllus infantis* CECT 30759 for fixing nitrogen.

Also, in the present example *Bacilllus infantis* CECT 30759 was applied to lettuce plant and tested with commercial product based on biological fixing nitrogen and organic fertilizer (70% Nitrogen) as positive control.

The assay was performed in a plant grown up in a plot (1 L) yellow peat with fertilizer, applying by spray on

phylllosphere 5 repetitions of each treatment (*Bacilllus infantis* CECT 30759, commercial product and organic fertilizer). The plant was treated the needed time to colonize the leave by microorganism.

The results were measured for height, fresh weight (FW) and dry weight (DW) and analyzed by the one-way ANOVA used to compare the means of more than two groups, when there is one independent variable and one dependent variable.

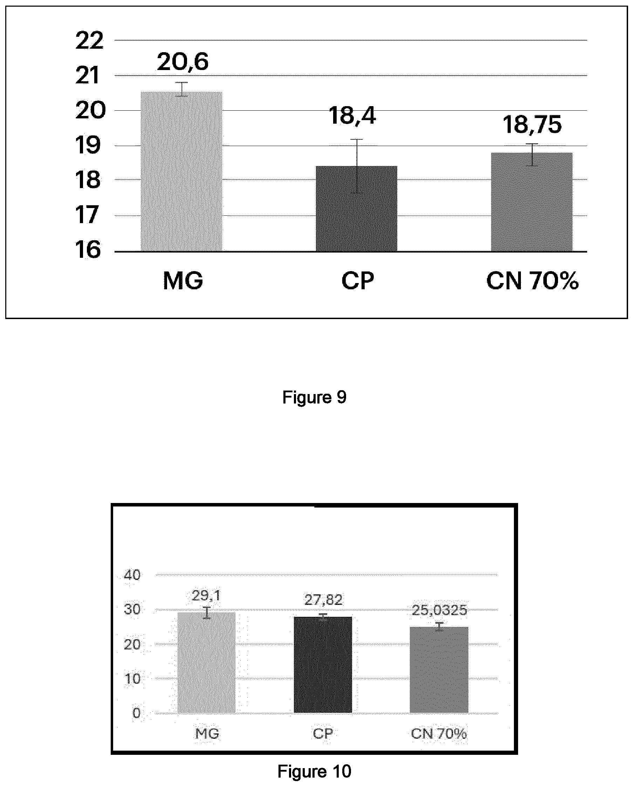

The phyllosphere treated with *Bacilllus infantis* CECT 30759 was higher than treated with commercial product or organic fertilizer **(Figure 9)**.

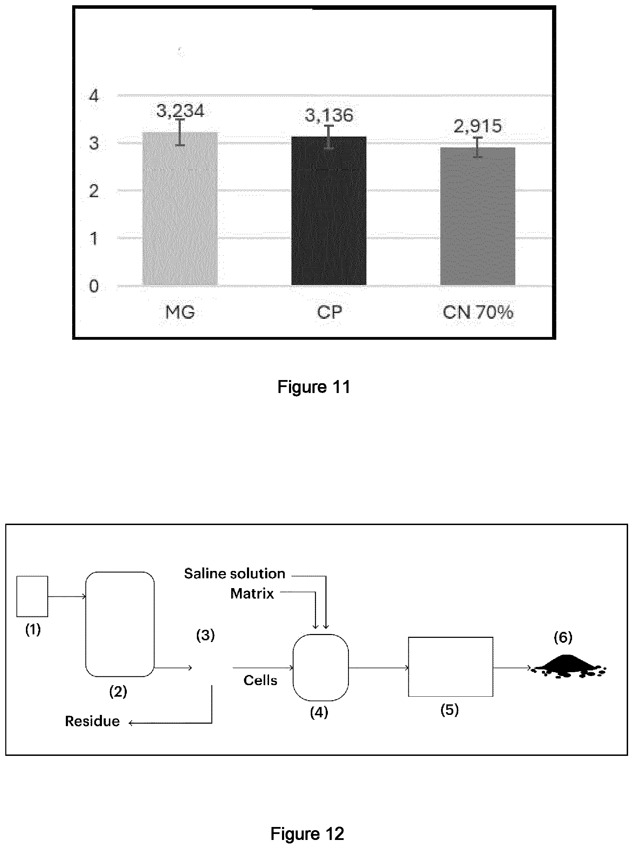

Additionally, the phyllosphere treated with *Bacilllus infantis* CECT 30759 was heavier than treated with commercial product or the organic fertilizer, fresh and dry weight **(Figure 10) (Figure 11)**.

## Example 4: Process to manufacture the microbial composition

One embodiment of the present invention, the microbial composition claimed is made up of *Bacilllus infantis* CECT 30759, and as example of microorganisms used in agriculture: *M. aquaticum* and *M. aminovorans.*

Each strain is cultured independently in the following culture medium: Sucrose (20 g/L), asparagine (2 g/L), K2HPO4 (1,31 g/L) and MgSO4 (1M - 4,2 mL/L), at 28°C minimum 24h.

Each strain is grown as **Figure 12** is shown; First step, a cultured strain is inoculated in a (1) starter 1 %, at 28°C for 48 hours. Following, the culture is transferred to bioreactor (2) and is grown at 28 °C for 48 hours. The result of fermentation is centrifuged (3) at 350l/h, separating the liquid and the solid (cells) is formulated (4) with (Saline solution) 15% Phosphate-buffered saline, (Matrix) 5% carboxymethyl cellulose and 0,5% Xanthan gum. The solid is dried at 30°C for 24 hours (4), posteriorly it is milled and pulverized (5), obtaining a bacterial powder.

One example of the formulation final might be $10^7$ CUF/g of each microorganism, 30 % fulvic acids, 10% CSL, humectant, cyclodextrin and dispersants adding up to 100% of composition.

## Example 5: Microbial composition for agricultural use

The final microbial composition of example 1 in the present invention applied to corn plant. The microbial composition was tested as example 3 *Bacilllus infantis* CECT 30759 isolated, this was applied to corn plant and tested with commercial product based on biological fixing nitrogen, organic fertilizer (70% Nitrogen) and organic fertilizer (100% Nitrogen) both as positive control.

The assay was performed in a plant grown up in a plot (7 L) yellow peat with fertilizer, applying by spray on phyllosphere, 8 repetitions of each treatment: Microbial composition, commercial product, 70% N and 100%N organic fertilizers. The plant was treated the needed time to colonize the leave by microorganism.

The results were measured for height, fresh weight (FW) and dry weight (DW) and analyzed by the one-way ANOVA.

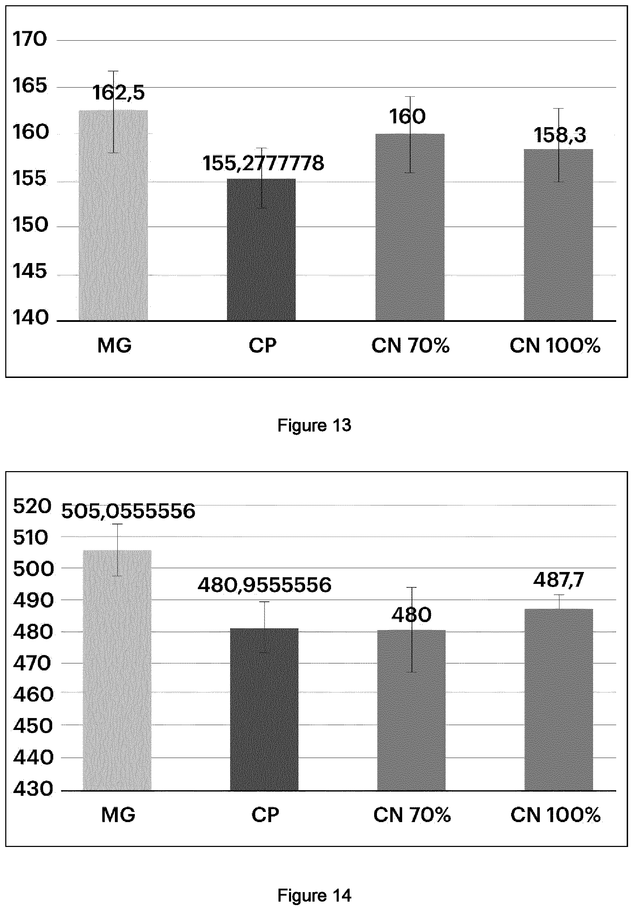

The phyllosphere treated with microbial composition was higher than treated with commercial product,70% N and 100%N organic fertilizers **(Figure 13)**.

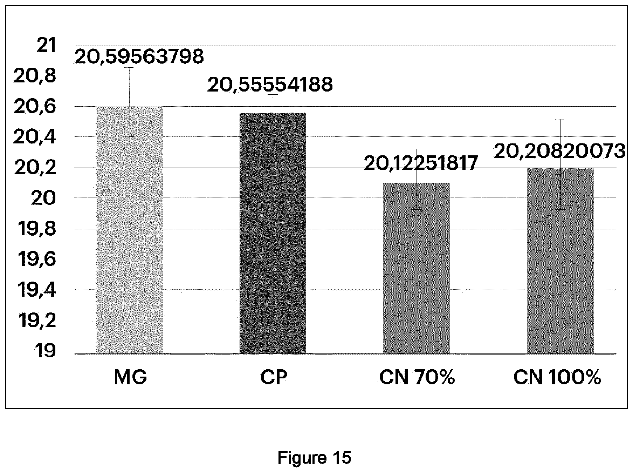

The phyllosphere treated with microbial composition was heavier than treated with commercial product and organic fertilizers regard to fresh weight **(Figure 14)** and % fresh weight/ dry weight **(Figure 15)**.

## Example 6: Method for reducing the need of nitrogen in crops

The final microbial composition of example 1 in the present invention was applied to different crops. The issue is to reduce the current requirement of nitrogen fertilizer in crops such as is represented in the following field trials.

The microbial composition (MC) was compared with commercial product applied to fixing atmospheric nitrogen (CP) based on microorganisms, and organic fertilizers (60%N, 70%N and 100%N) no treated.

The field trials were designed to reduce the amount of nitrogen fertilizer up to 60% of the total required by each crop. The Nitrogen Use Efficiency (NUE) is related to productivity is calculated by following mathematical formula:

$$Nf = (Na/ef) - (Ni + Nmin)$$

where:

- **Nf**: Nitrogen Fertilizer applied at the crop system (Kg)
- **Na**: Nitrogen Uptake by plant (kg) = Goal Productivity (T grain/ ha)* Nitrogen requirement coefficient (Kg N/ ha)
- **ef**: Use efficiency (standard coefficient to simplify the mass balance between uptake and returning of Nitrogen to system)
- **Ni**: Initial Nitrogen amount (kg)

- **Nmin**: Nitrogen mineralized from the Organic Matter (g)

Wheat Crop

Wheat is a grass widely cultivated for its seed, a cereal grain that is a staple food around the world. The many species of wheat together make up the genus *Triticum* the most widely grown is common wheat (*T. aestivum*).

Wheat is grown on a larger area of land than any other food crop (220.7 million hectares or 545 million acres in 2021). World trade in wheat is greater than for all other crops combined. In 2021, world wheat production was 771 million tons (850 million short tons), making it the second most-produced cereal after maize.

The microbial composition in the present invention (MC) was applied at tillering of wheat, doses 200-g/ha. The commercial product (CP) was applied to according the recommended doses in the label.

**TABLE 2: Field trial for wheat.**

| Composition | Nitrogen reduction | Yield kg/ha | Improvement on yield vs C-100% N | Improvement on yield vs C- 70% N | Improvement on yield vs C- 60% N |
|---|---|---|---|---|---|
| UNTREATED 100%N | | 1.768,89 | | 12,75% | 32,67% |
| UNTREATED 70%N | -30% | 1.568,89 | -11,31% | | 17,67% |
| UNTREATED 60%N | -40% | 1.333,33 | -24,62% | -15,01% | |
| MC | -40% | 1.728,89 | -2,26% | 10,20% | 29,67% |
| CP | -40% | 1.466,67 | -17,09% | -6,52% | 10,00% |

Corn Crop

Maize (Zea mays), also known as corn in North American, is a tall stout grass that produces cereal grain. Maize is cultivated throughout the world; a greater weight of maize is produced each year than any other grain. In 2020, world production was 1.1 billion tons. Modern plant breeding has greatly increased output and qualities such as nutrition, drought tolerance, and tolerance of pests and diseases.

In the present invention microbial composition (MC) was applied at tillering, doses 200 g/ha at the time of peak vegetative development. Commercial product (CP) was applied to according the recommended doses in the label.

**TABLE 3: Field trial for corn.**

| Composition | Nitrogen reduction | Yield kg/ha | Improvement on yield vs C-100% N | Improvemen t on yield vs C- 70% N | Improvement on yield vs C-60% N |
|---|---|---|---|---|---|
| UNTREATED 100%N | | 15.960 | | -7,64% | -9,66% |
| UNTREATED 70%N | 30% | 17.280 | 8,27% | | -2,19% |
| UNTREATED 60%N | 40% | 17.666,67 | 10,69% | 2,24% | |
| MC | -30% | 16.413,33 | 2,84% | -5,02% | -7,09% |
| CP | -30% | 15.280 | -4,26% | -11,57% | -13,51% |

Olive trees

The olive, botanical name *Olea europaea,* is a species of small tree or shrub in the family Oleaceae, found traditionally in the Mediterranean Basin.

Another example for reducing the requirement of nitrogen fertilizer in trees, the microbial composition (MC) was applied at shoot growing and doses 200-g/ha. Commercial product (CP) was applied to according the recommended doses in the label.

**TABLE 4: Field trial for olive tree .**

| Composition | Nitrogen reduction | Yield kg/ha | Improvement on yield vs C-100% N | Improvement on yield vs C-70% N | Improvement on yield vs C-60% N |
|---|---|---|---|---|---|
| UNTREATED 100%N | | 3.314,32 | | 5,25% | 7,28% |
| UNTREATED 70%N | | 3.148,89 | -4,99% | | 1,92% |
| UNTREATED 60%N | | 3.089,44 | -6,78% | -1,89% | |
| MC | -30% | 3.398,89 | 2,55% | 7,94% | 10,02% |
| MC | -40% | 3.565,80 | 7,59% | 13,24% | 15,42% |
| CP | -30% | 3.530,37 | 6,52% | 12,11% | 14,27% |
| CP | -40% | 3.218,02 | -2,91% | 2,20% | 4,16% |

As a result, the microbial composition can be considered for biostimulant application, providing resilience to different crops against biotic and abiotic stress. Also, the microbial composition showed the capacity for reducing the current requirement of nitrogen fertilizer in crops.

**References**

This list of references cited by the applicant is for the readers convenience only. It does not form part of the European patent document. Even though great care has been taken in compiling the references, errors or omissions cannot be excluded and the EPO disclaims all liability in this regard.

**Patent documents cited in the description**

- EP0376853 "Homogeneous granular nitrogen fertilizer" 28.12.1989.
- EP4247775 "Nitrogen fertilizer compositions based on polyphosphate caged structure" 21.11.2021.
- EP1379558 "A urea-based nitrogenous fertilizer coated with zeolite" 10.04.2002.
- CN101565125 "Sequencing feeding device" 21.04.2008.
- EP4330263 "Method for producing phosphoryl or thiophosphoryl triamide, and use of compound in nitrogen fertilizer formulations" 06.03.2024.
- EP1114009 "Highly available particulate controlled release nitrogen fertilizer" 05.08.1999.
- US9,580,363 "Nitrogen-fixing bacterial inoculant for improvement of crop productivity and reduction of nitrous oxide emission" 20.03.2013.
- CN106085898 "Bacillus subterraneus and biological control preparation comprising same and application of biological control preparation" 03.06.2016.
- CN107164279" Composite microbial agent for promoting growth of crops, and application thereof" 26.06.2017.

**Non-patent literature cited in the description**

- Zayed O, Hewedy OA, Abdelmoteleb A, Ali M, Youssef MS, Roumia AF, Seymour D, Yuan ZC. Nitrogen Journey in Plants: From Uptake to Metabolism, Stress Response, and Microbe Interaction. Biomolecules. 2023 Sep 25;13(10):1443.
- Estruch JJ, Warren GW, Mullins MA, Nye GJ, Craig JA, Koziel MG. Vip3A, a novel Bacillus thuringiensis vegetative insecticidal protein with a wide spectrum of activities against lepidopteran insects. Proc Natl Acad Sci U S A. 1996 May 28;93(11):5389-94.
- Fürnkranz, M., Wanek, W., Richter, A. et al. Nitrogen fixation by phyllosphere bacteria associated with higher plants and their colonizing epiphytes of a tropical lowland rainforest of Costa Rica. ISME J 2, 561-570 (2008)
- Solanki MK, Wang Z, Wang FY, Li CN, Gupta CL, Singh RK, Malviya MK, Singh P, Yang LT, Li YR. Assessment of Diazotrophic Proteobacteria in Sugarcane Rhizosphere When Intercropped With Legumes (Peanut and Soybean) in the Field. Front Microbiol. 2020 Jul 31;11:1814.

- Krogmeier MJ, McCarty GW, Bremner JM. Phytotoxicity of foliar-applied urea. Proc Natl Acad Sci U S A. 1989 Nov;86(21):8189-91.
- Liu XX, Zhu YX, Fang XZ, Ye JY, Du WX, Zhu QY, Lin XY, Jin CW. Ammonium aggravates salt stress in plants by entrapping them in a chloride over-accumulation state in an NRT1.1-dependent manner. Sci Total Environ. 2020 Dec 1;746:141244
- Lowell L. Trent, Rue S. Hestand III and Chris C. Carter. Toxic of sulfuric acid to aquatic plants and organisms. J.Aquat, Plant Manage.16:1978.
- Ankita,A., Anandita,D., Arun Prasad,A.S. and Bhaskara Rao,K.V. Biodegradation of textile azo dye. Journal School of Biosciences and Technology, Vellore Institute of Technology University.2012.
- Girish,C., wani,S.P., S,G., Ankita,M., Swati,C., Pawar,C., Manoj,K. and Kesevarao,A. Microbial consortium culture and vermi composting technologies for recycling on-farm wastes and food production. Journal Biocontrol, icrisat, patancheru, hyderabad, telanaga 502324, India. 2018.
- Sun,X., Zheng,P., Wan,Q. and Shen,J. Algae-lysing characteristics of three algicidal bacteria against Anabaena flos-aquae. JOURNAL Dept. of Environmental Science and Resource, School of Agriculture and Life Science, Shanghai Jiaotong University, 800 Dongchuan Rd. Minhang District, Shanghai 200240, China. 2012.
- Dahal, R.H. Strains related to functional cosmetics. Journal Life Science, Kyonggi University, Kyonggi University, Suwon, Gyeonggi-Do 16227, South Korea. 2019.
- Volova,T.G., Boyandin,A.N., Vasil'ev,A.D., Karpov,V.A., Kozhevnikov,I.V., Prudnikova,S.V., Rudnev,V.P., Xuan,B.B., Dung,V.T. and Gitel'zon,I.I. Biodegradation of Polyhydroxyalkanoates (PHAs) in the South China Sea and Identification of PHA-degrading Bacteria. Journal Microbiology 80 (2), 252-260. 2011.
- Tang,Q. and Liu,Z.X. Isolation and Identification of Siderophore-producing Bacteria in Sediments from Tidal Zone of Naozhou Island. Journal College of Bio-resources and Environmental Science, Jishou University, Renmin Road, Jishou 416000, China. 2021.
- Rodrigo,W.W.P., Hemachandra,C.K., Iddamalgoda, H.K.S.P.S. and Fonseka, W.R.K. Molecular characterization of crude oil degrading bacterial strains isolated from petroleum contaminated sites in Sri Lanka. Journal Biotechnology Unit, Industrial Technology Institute, 363, Bauddhaloka Mawatha, Colombo, Western 00700, Sri Lanka. 2017.
- Desai,C., Parikh,R.Y., Vaishnav,T., Shouche,Y.S. and Madamwar,D. Tracking the influence of long-term chromium pollution on soil bacterial community structures by comparative analyses of 16S rRNA gene phylotypes Journal Res. Microbiol. (2008).
- Tran,L.H. Potential antagonistic ability against pathogenic nematode of endophytic bacteria isolated from coffee roots in Vietnam. Journal key laboratory, institute of biotechnology, 18 hoang quoc viet, ha noi 100000, Viet Nam.2018
- Khaneja,R., Perez-Fons,L., Fakhry,S., Baccigalupi,L., Steiger,S.,To,E., Sandmann,G., Dong,T.C., Ricca,E., Fraser,P.D. and Cutting,S.M. Carotenoids found in Bacillus J. Appl. Microbiol. (2009).
- Reis VM, Teixeira KR. Nitrogen fixing bacteria in the family Acetobacteraceae and their role in agriculture. J Basic Microbiol. 2015 Aug;55(8):931-49.
- Mieszkin S, Pouder E, Uroz S, Simon-Colin C, Alain K. Acidisoma silvae sp. nov. and Acidisomacellulosilytica sp. nov., Two Acidophilic Bacteria Isolated from Decaying Wood, Hydrolyzing Cellulose and Producing Poly-3-hydroxybutyrate. Microorganisms. 2021 Sep 28;9(10):2053.
- Antoszewski M, Mierek-Adamska A, Dąbrowska GB. The Importance of Microorganisms for Sustainable Agriculture-A Review. Metabolites. 2022 Nov 11;12(11):1100.
- Sanket Ray. Beneficial Microbes in Agro-Ecology, 2020.
- Patrick du Jardin. Plant biostimulants: Definition, concept, main categories and regulation, Scientia Horticulturae, Volume 196, 2015, Pages 3-14, ISSN 0304-4238.
- Mushtaq H, Ganai SA, Jehangir A, Ganai BA, Dar R. Molecular and functional characterization of protease from psychrotrophic Bacillus sp. HM49 in North-western Himalaya. PLoS One. 2023 Mar 30;18(3):e0283677.
- Sethi S, Datta A, Gupta BL, Gupta S. Optimization of cellulase production from bacteria isolated from soil. ISRN Biotechnol. 2013 Feb 19;2013:985685.
- Souza Rd, Ambrosini A, Passaglia LM. Plant growth-promoting bacteria as inoculants in agricultural soils. Genet Mol Biol. 2015 Dec;38(4):401-19.
- Walter V, Syldatk C, Hausmann R. Screening Concepts for the Isolation of Biosurfactant Producing Microorganisms. In: Madame Curie Bioscience Database [Internet]. Austin (TX): Landes Bioscience; 2000-2013.
- Castellano-Hinojosa, A., y Bedmar, E.J. "Methods for evaluating Plant Growth-promoting rhizobacteria traits", in Advances in PDPR Research, ed. (2017).

**Claims**

1. A strain of *Bacillus infantis* deposited under number CECT 30759 **characterized by** promoting the growth in plant, producing biosurfactants and by having capability of fixing nitrogen.

2. The strain of *Bacillus infantis* deposited under number CECT 30759 according to claim 1 having at least 97% sequence identity of SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

3. A microbial composition comprising:

   A strain of *Bacillus infantis* deposited under number CECT 30759;
   at least one microorganism used in agriculture; and
   one or more compounds used in formulation of agricultural products.

4. The microbial composition according to claim 3, wherein the microorganism used in agriculture is at least one strain selected from genus *Acetobacter sp, Acidisoma sp., Acinetobacter sp., Agrobacterium sp., Alternaria sp., Anabaena sp., Arthrobacter sp., Aspergillus sp., Aulosira sp., Azospirillum sp, Azotobacter sp., Bacillus sp., Bradyrhizobium sp., Brevibacillus sp., Burkholderia sp., Calothrix sp., Candida sp., Chlorella sp., Clitocybe sp., Clostridium sp, Collybia sp., Cyathus sp., Ensifer sp., Enterobacter sp., Frankia sp., Funneliformis sp, Fusarium sp., Glomus sp., Hapalosiphon sp., Hebeloma sp., Humicola sp., Laccaria sp., Lactobacillus sp. Lecanicillium sp., Metarhizium sp., Methylobacterium sp, Microcystis sp., Mortierella sp., Mucor sp., Nostoc sp., Oscillatoria sp, Paenibacillus sp., Pseudomonas sp., Penicillium sp., Phoma sp, Phormidium sp. Purpureocillium sp., Rhizobium sp., Rhodoferax sp., Scenedesmus sp, Serratia sp., Spirulina sp., Streptomyces sp. Tolypothrix sp., Trichoderma sp., and Virgibacillus sp.*

5. The microbial composition according to claim 4, wherein the microorganism used in agriculture is at least one specie selected from genus *Methylobacterium*

6. The microbial composition according to claim 5, wherein the microorganism used in agriculture belong to the species *Methylobacterium aquaticum* and *Methylobacterium aminovorans.*

7. The microbial composition according to claim 3, wherein the microbial concentration at least $10^7$CFU/g by each microorganism thereof.

8. The microbial composition according to claim 3, wherein the compounds used in formulation comprises one compound or a combination thereof selected from the group consisting of adjuvants, alginic acid, amino acids, arabinogalactan, bioplastic, carbohydrates, carboxymethyl cellulose, carboxylic acids, cellulose, chelated minerals, chitosan, complex minerals, cyclodextrin, disaccharides, dispersants, fertilizer mixtures, fulvic acids, arabic gum, humectant, humic acids, hydrogel, hydrolysates, hydrolyzed proteins, lignosulfonates, macronutrients, maltodextrins, maltodextrose, metal ions, methoxyl pectin, micronutrients, monosaccharides, organic acids, peptides, proteases, polyacrylic acid, salts, secondary-nutrients, silica, silver, sodium alginate, sodium selenosulfate, surfactants, titanium dioxide, UV protectants, vitamins, wetting agents, xanthan gum and zinc oxide.

9. A process for the preparation of a microbial composition as defined in claim 3 comprising the steps of:

   growing of microorganisms independently in a proper culture medium;
   fermenting the microorganisms;
   separating the fermented mixture in residual liquid and cells;
   mixing the bacterial cells, saline solution and a matrix;
   drying to obtain a bacterial powder; and
   adding compounds to obtain the final microbial composition.

10. Use of the microbial composition according to claim 3 applied by spraying to the phyllosphere of the plant.

11. Use of the microbial composition according to claim 3, **characterized by** improving mineral nutrition in plant.

12. Use of the microbial composition according to claim 3, **characterized by** promoting the growth of crops.

13. Use of the microbial composition according to claim 3, **characterized by** being used as a biostimulant for crops.

14. Use of the microbial composition according to claim 3, wherein composition is a solid formulation.

15. Use of the microbial composition according to claim 14, wherein composition is preferably applied as powder.

16. Use of the microbial composition according to claim 14, applied alone or in combination with additional components such as liquid fertilizer, amino acids and other products and techniques used in agriculture.

17. Method for improving mineral nutrition and promoting the growth in plant **characterized by** applying the microbial composition according to claim 5 reducing the total amount of nitrogen fertilizer required by crop between (30-60) %.

18. Method for improving mineral nutrition and promoting the growth in plant according to claim 17 **characterized by** reducing the total amount of Nitrogen Fertilizer required by crop between (30-40) %.

(a)                    (b)                    (c)

Figure 1

Figure 2

Figure 3

Figure 4

Figure 5

(a)                                          (b)

Figure 6

Figure 7

Figure 8

Figure 9

Figure 10

**Figure 11**

**Figure 12**

Figure 13

Figure 14

Figure 15

| | Europäisches Patentamt European Patent Office Office européen des brevets | **EUROPEAN SEARCH REPORT** | **Application Number** EP 24 38 3080 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOWHAN LAVUDYA BINDU ET AL: "Plant growth promoting and antagonistic traits of bacteria isolated from forest soil samples", IRANIAN JOURNAL OF MICROBIOLOGY, 17 April 2023 (2023-04-17), XP093256491, IR ISSN: 2008-3289, DOI: 10.18502/ijm.v15i2.12480 * abstract * * table 2 * | 1-18 | INV. A01N63/22 A01P21/00 C12N1/20 |
| A | Tshishonga Khuthadzo: "The genomic profiles of endophytic bacteria isolated from Pellaea calomelanos" In: "The genomic profiles of endophytic bacteria isolated from Pellaea calomelanos", 22 August 2017 (2017-08-22), University of Johannesburg, Johannesburg [SA], XP093256631, pages 1-194, Retrieved from the Internet: URL:https://ujcontent.uj.ac.za/esploro/out puts/graduate/The-genomic-profiles-of-endo phytic-bacteria/9913341907691> * abstract * * 1.1 Introduction; page 2 * | 1-18 | |
| A | US 2020/187507 A1 (HELD DAVID W [US] ET AL) 18 June 2020 (2020-06-18) * claims * | 1-18 | TECHNICAL FIELDS SEARCHED (IPC) C12R A01N A01P C12N |
| | -/-- | | |

The present search report has been drawn up for all claims

| Place of search | Date of completion of the search | Examiner |
|---|---|---|
| Munich | 6 March 2025 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 38 3080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YOUSUF JESMI ET AL: "Nitrogen fixing potential of various heterotrophic Bacillus strains from a tropical estuary and adjacent coastal regions", JOURNAL OF BASIC MICROBIOLOGY, vol. 57, no. 11, 8 August 2017 (2017-08-08), pages 922-932, XP093256627, BERLIN, DE ISSN: 0233-111X, DOI: 10.1002/jobm.201700072 Retrieved from the Internet: URL:https://onlinelibrary.wiley.com/doi/pdfdirect/10.1002/jobm.201700072> * abstract * ----- | 1-18 | |
| | | | TECHNICAL FIELDS SEARCHED (IPC) |

The present search report has been drawn up for all claims

| Place of search | Date of completion of the search | Examiner |
|---|---|---|
| Munich | 6 March 2025 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 3080

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2025

| Patent document cited in search report | Publication date | Patent family member(s) | Publication date |
|---|---|---|---|
| US 2020187507 A1 | 18-06-2020 | NONE | |

EPO FORM P0459

For more details about this annex : see Official Journal of the European Patent Office, No. 12/82

## REFERENCES CITED IN THE DESCRIPTION

### Patent documents cited in the description

- EP 0376853 A
- EP 4247775 A
- EP 1379558 A
- CN 101565125
- EP 4330263 A
- EP 1114009 A
- US 9580363 B
- CN 106085898
- CN 107164279
- KR 1020220162897
- WO 9717432 A
- WO 9808932 A
- WO 200147952 A
- WO 9850427 A
- WO 2009152359 A
- WO 2007024782 A
- US 6855533 B
- WO 2019103918 A
- WO 2002040677 A
- WO 2006128569 A
- WO 2006128570 A
- US 2002120964 A
- WO 2002034946 A
- WO 2010117737 A
- WO 2010117735 A
- WO 2005103266 A
- US 2005216969 A
- US 2007143876 A
- WO 2006098952 A
- US 200635230473 A
- WO 2002027004 A
- WO 11075593 A
- WO 2011075595 A
- WO 2010077816 A
- US 2006162007 A
- WO 2004053062 A
- US 2003126634 A
- WO 2010080829 A
- WO 2005074671 A
- US 2009217423 A
- WO 2006128573 A
- US 20100024077 A
- WO 2006128571 A
- WO 2006128572 A
- US 2006130175 A
- WO 2004039986 A
- US 2007067868 A
- WO 2005054479 A
- WO 2005054480 A
- WO 2012033794 A
- WO 2011022469 A
- WO 2012075426 A
- WO 2012075429 A
- US 2006070139 A
- WO 2009100188 A
- WO 2011066384 A
- WO 2011066360 A
- US 200930137395 A
- WO 08112019 A
- US 2008312082 A
- WO 2008054747 A
- US 20090210970 A
- WO 2009103049 A
- US 20100184079 A
- WO 2008002872 A
- WO 07091277 A
- US 2006059581 A
- WO 98044140 A
- WO 2011063413 A
- US 2005086719 A
- US 2005188434 A
- WO 2008151780 A
- US 2010050282 A
- WO 2007017186 A
- WO 2010076212 A
- US 2004172669 A
- WO 2004074492 A
- US 2008064032 A
- WO 2006108674 A
- US 2008320616 A
- WO 2006108675 A
- US 2008196127 A
- WO 2003013224 A
- US 2003097687 A
- US 6468747 B
- WO 2000026345 A
- US 20082289060 A
- WO 2000026356 A
- US 2007028322 A
- WO 2005061720 A
- US 2009300784 A
- WO 2007142840 A
- WO 2005103301 A
- US 2004250317 A
- WO 2002100163 A
- US 2002102582 A
- WO 2004011601 A
- US 2006095986 A

- WO 2011062904 A
- WO 2009111263 A
- US 20110138504 A
- US 2009130071 A
- WO 2009064652 A
- US 20100080887 A
- WO 2010037016 A
- WO 2011034704 A
- WO 2012051199 A
- WO 2010024976 A
- US 20110067141 A
- WO 2009102873 A
- US 2008028482 A
- WO 2005059103 A
- WO 2004072235 A
- US 2006059590 A
- WO 2011153186 A
- WO 2012134808 A
- WO 07140256 A
- US 2008260932 A
- US 2006282915 A
- WO 2006130436 A
- WO 2001031042 A
- WO 2001041558 A
- US 2003188347 A
- US 2007292854 A
- WO 2008114282 A
- US 200320188347 A
- WO 2002036831 A
- US 2008070260 A
- WO 2012082548 A
- WO 200244407 A

- US 2009265817 A
- US 2001029014 A
- WO 2001051654 A
- US 2010077501 A
- WO 2008122406 A
- WO 2006128568 A
- US 2005039226 A
- WO 2004099447 A
- WO 2003052073 A
- WO 2011084632 A
- WO 2011084621 A
- WO 2011063413 A2
- WO 2011066360 A1
- WO 2011066384 A1
- WO 2011075593 A1
- WO 2011075595 A1
- WO 2011084621 A1
- WO 2011084632AI A
- WO 2011153186 A1
- WO 2012033794 A2
- WO 2012051199 A2
- WO 2012075426AI A
- WO 2012075429 A1
- WO 2012082548 A2
- WO 2012071039 A1
- US 2012131692 A
- WO 2012075426 A2
- WO 2012075429 A2
- WO 2012134808 A1
- WO 2013003558 A1
- WO 2013010094 A1
- WO 2013012775 A1

**Non-patent literature cited in the description**

- **ESTRUCH et al.** *Proc Natl Acad Sci US A.*, 1996, vol. 28;93 (11), 5389-94
- **ZAYED O** ; **HEWEDY OA** ; **ABDELMOTELEB A** ; **ALI M** ; **YOUSSEF MS** ; **ROUMIA AF** ; **SEYMOUR D** ; **YUAN ZC**. Nitrogen Journey in Plants: From Uptake to Metabolism, Stress Response, and Microbe Interaction. *Biomolecules*, 25 September 2023, vol. 13 (10), 1443
- **ESTRUCH JJ** ; **WARREN GW** ; **MULLINS MA** ; **NYE GJ** ; **CRAIG JA** ; **KOZIEL MG**. Vip3A, a novel Bacillus thuringiensis vegetative insecticidal protein with a wide spectrum of activities against lepidopteran insects. *Proc Natl Acad Sci U S A*, 28 May 1996, vol. 93 (11), 5389-94
- **FÜRNKRANZ, M** ; **WANEK, W** ; **RICHTER, A et al.** Nitrogen fixation by phyllosphere bacteria associated with higher plants and their colonizing epiphytes of a tropical lowland rainforest of Costa Rica. *ISME J*, 2008, vol. 2, 561-570

- **SOLANKI MK** ; **WANG Z** ; **WANG FY** ; **LI CN** ; **GUPTA CL** ; **SINGH RK** ; **MALVIYA MK** ; **SINGH P** ; **YANG LT** ; **LI YR**. Assessment of Diazotrophic Proteobacteria in Sugarcane Rhizosphere When Intercropped With Legumes (Peanut and Soybean) in the Field. *Front Microbiol*, 31 July 2020, vol. 11, 1814
- **KROGMEIER MJ** ; **MCCARTY GW** ; **BREMNER JM**. Phytotoxicity of foliar-applied urea. *Proc Natl Acad Sci U S A.*, November 1989, vol. 86 (21), 8189-91
- **LIU XX** ; **ZHU YX** ; **FANG XZ** ; **YE JY** ; **DU WX** ; **ZHU QY** ; **LIN XY** ; **JIN CW**. Ammonium aggravates salt stress in plants by entrapping them in a chloride over-accumulation state in an NRT1.1-dependent manner. *Sci Total Environ*, 01 December 2020, vol. 746, 141244
- **LOWELL L. TRENT** ; **RUE S. HESTAND III AND CHRIS C. CARTER**. Toxic of sulfuric acid to aquatic plants and organisms. *J.Aquat, Plant Manage*, vol. 16, 1978

- **ANKITA,A** ; **ANANDITA,D** ; **ARUN PRASAD,A.S** ; **BHASKARA RAO**. K.V. Biodegradation of textile azo dye. Journal School of Biosciences and Technology. *Vellore Institute of Technology University*, 2012
- **GIRISH,C** ; **WANI,S.P., S,G** ; **ANKITA,M** ; **SWATI,C.** ; **PAWAR,C** ; **MANOJ,K** ; **KESEVARAO,A**. Microbial consortium culture and vermi composting technologies for recycling on-farm wastes and food production. *Journal Biocontrol, icrisat, patancheru, hyderabad, telanaga 502324, India*, 2018
- Algae-lysing characteristics of three algicidal bacteria against Anabaena flos-aquae. **SUN,X.** ; **ZHENG,P.** ; **WAN,Q** ; **SHEN,J**. JOURNAL Dept. of Environmental Science and Resource. School of Agriculture and Life Science, Shanghai Jiaotong University, 2012
- Strains related to functional cosmetics. **DAHAL, R.H**. Journal Life Science. Kyonggi University, Kyonggi University, 2019
- **VOLOVA,T.G.** ; **BOYANDIN,A.N.** ; **VASIL'EV,A.D.** ; **KARPOV,V.A.** ; **KOZHEVNIKOV,I.V.** ; **PRUDNIKOVA,S.V.** ; **RUDNEV,V.P.** ; **XUAN,B.B.** ; **DUNG,V.T.** ; **GITEL'ZON,I.I.** Biodegradation of Polyhydroxyalkanoates (PHAs) in the South China Sea and Identification of PHA-degrading Bacteria. *Journal Microbiology*, 2011, vol. 80 (2), 252-260
- Isolation and Identification of Siderophore-producing Bacteria in Sediments from Tidal Zone of Naozhou Island. **TANG,Q** ; **LIU,Z.X**. Journal College of Bio-resources and Environmental Science. Jishou University, 2021
- W.R.K. Molecular characterization of crude oil degrading bacterial strains isolated from petroleum contaminated sites in Sri Lanka. **RODRIGO,W.W.P** ; **HEMACHANDRA,C.K.** ; **IDDAMALGODA, H.K.S.P.S** ; **FONSEKA**. Journal Biotechnology Unit. Industrial Technology Institute, 2017
- **DESAI,C** ; **PARIKH,R.Y.** ; **VAISHNAV,T** ; **SHOUCHE,Y.S** ; **MADAMWAR,D**. Tracking the influence of long-term chromium pollution on soil bacterial community structures by comparative analyses of 16S rRNA gene phylotypes Journal Res. *Microbiol*, 2008
- Potential antagonistic ability against pathogenic nematode of endophytic bacteria isolated from coffee roots in Vietnam. **TRAN,L.H**. Journal key laboratory. institute of biotechnology, 2018
- **KHANEJA,R** ; **PEREZ-FONS,L** ; **FAKHRY,S** ; **BACCIGALUPI,L** ; **STEIGER,S.** ; **TO,E** ; **SANDMANN,G** ; **DONG,T.C** ; **RICCA,E** ; **FRASER,P.D**. Carotenoids found in Bacillus. *J. Appl. Microbiol*, 2009
- **REIS VM** ; **TEIXEIRA KR**. Nitrogen fixing bacteria in the family Acetobacteraceae and their role in agriculture. *J Basic Microbiol*, 2015, vol. 55 (8), 931-49
- **MIESZKIN S** ; **POUDER E** ; **UROZ S** ; **SIMON-COLIN C** ; **ALAIN K**. Acidisoma silvae sp. nov. and Acidisomacellulosilytica sp. nov., Two Acidophilic Bacteria Isolated from Decaying Wood, Hydrolyzing Cellulose and Producing Poly-3-hydroxybutyrate. *Microorganisms*, 28 September 2021, vol. 9 (10), 2053
- **SANKET RAY**. *Beneficial Microbes in Agro-Ecology*, 2020
- **PATRICK DU JARDIN**. Plant biostimulants: Definition, concept, main categories and regulation. *Scientia Horticulturae*, 2015, vol. 196, ISSN 0304-4238, 3-14
- **MUSHTAQ H** ; **GANAI SA** ; **JEHANGIR A** ; **GANAI BA** ; **DAR R**. Molecular and functional characterization of protease from psychrotrophic Bacillus sp. HM49 in North-western Himalaya. *PLoS One*, 30 March 2023, vol. 18 (3), e0283677
- **SETHI S** ; **DATTA A** ; **GUPTA BL** ; **GUPTA S**. Optimization of cellulase production from bacteria isolated from soil. *ISRN Biotechnol*, 19 February 2013, 985685
- **SOUZA RD** ; **AMBROSINI A** ; **PASSAGLIA LM**. Plant growth-promoting bacteria as inoculants in agricultural soils. *Genet Mol Biol*, 2015, vol. 38 (4), 401-19
- Screening Concepts for the Isolation of Biosurfactant Producing Microorganisms. **WALTER V** ; **SYLDATK C** ; **HAUSMANN R**. Madame Curie Bioscience Database [Internet. Landes Bioscience, 2000
- Methods for evaluating Plant Growth-promoting rhizobacteria traits. **CASTELLANO-HINOJOSA, A.** ; **Y BEDMAR, E.J**. Advances in PDPR Research. 2017